# EUROPEAN PATENT APPLICATION

(11) **EP 4 714 945 A1**
(43) Date of publication of application: **25.03.2026**
(21) Application number: 24839952.9
(22) Date of filing: 27.06.2024
(51) Int. Cl.: C07D 307/91, C07D 333/76, C07D 409/12, H10K 85/60

(54) **COMPOUND FOR ORGANIC ELECTRIC ELEMENT, ORGANIC ELECTRIC ELEMENT USING SAME, AND ELECTRONIC DEVICE HAVING SAME**

(30) Priority: 10.07.2023 KR 20230089226
(71) Applicant: Duk San Neolux Co., Ltd., Chungcheongnam-do 31027 (KR)
(72) Inventor: LEE, Hee Chai, Cheonan-si, Chungcheongnam-do 31027 (KR); SONG, Hyeng Gun, Cheonan-si, Chungcheongnam-do 31027 (KR); LEE, Hyung Dong, Cheonan-si, Chungcheongnam-do 31027 (KR)
(74) Representative: Isarpatent
(86) International application number: PCT/KR2024/009007
(87) International publication number: WO 2025/014138

(57) **Abstract**

The present invention provides a novel compound that can improve the luminous efficiency, stability, and lifespan of the element, a composition comprising the same and an organic electronic element using the same, and an electronic device thereof.

## Description

### BACKGROUND

### [Technical Field]

The present invention relates to compounds for organic electronic elements, organic electronic elements using the same, and an electronic device thereof.

### [Background Art]

In general, organic light emitting phenomenon refers to a phenomenon that converts electric energy into light energy by using an organic material. An organic electronic element using an organic light emitting phenomenon usually has a structure including an anode, a cathode, and an organic material layer interposed therebetween. Here, in order to increase the efficiency and stability of the organic electronic element, the organic material layer is often composed of a multi-layered structure composed of different materials, and for example, may include a hole injection layer, a hole transport layer, an emitting layer, an electron transport layer, an electron injection layer etc.

A material used as an organic material layer in an organic electronic element may be classified into a light emitting material and a charge transport material, such as a hole injection material, a hole transport material, an electron transport material, an electron injection material etc. depending on its function.

The biggest issues with organic light emitting devices are their lifespan and efficiency, and as displays become larger in size, these efficiency and lifespan issues must be resolved.

Efficiency, lifespan, and driving voltage are interrelated. As efficiency increases, the driving voltage decreases relatively. As the driving voltage decreases, the crystallization of organic materials due to Joule heating generated during operation decreases, which results in a tendency for the lifespan to increase.

However, the efficiency cannot be maximized simply by improving the organic material layer. This is because, when the energy level and T1 value between each organic material layer, and the intrinsic properties (mobility, interfacial properties, etc.) of materials are optimally combined, long lifetime and high efficiency can be achieved at the same time.

Additionally, in order to solve the problem of light emission in the hole transport layer in recent organic light emitting devices, an emitting auxiliary layer must exist between the hole transport layer and the emitting layer, and it is time to develop different emitting auxiliary layers for each emitting layer (R, G, B). Typically, electrons are transferred from the electron transport layer to the emitting layer, and holes are transferred from the hole transport layer to the emitting layer, and excitons are generated through recombination.

However, since the material used in the hole transport layer must have a low HOMO value, most of them have a low T1 value, which causes excitons generated in the emitting layer to move to the hole transport layer, resulting in charge unbalance within the emitting layer and causing light emission at the interface of the hole transport layer.

When light is emitted at the interface of the hole transport layer, the color purity and efficiency of the organic electronic element are reduced, and its lifespan is shortened. Therefore, there is an urgent need to develop an emitting auxiliary layer with a high T1 value and a HOMO energy level between the HOMO energy level of the hole transport layer and the HOMO energy level of the emitting layer.

Meanwhile, it is necessary to develop a hole injection layer material that has stable properties, i.e., a high glass transition temperature, against Joule heating generated during device operation while delaying the penetration and diffusion of metal oxides from the anode electrode (ITO), which is one of the causes of shortened lifespan of organic electronic elements, into the organic layer. The low glass transition temperature of the hole transport layer material has been reported to significantly impact device lifespan by reducing the uniformity of the thin film surface during device operation. Furthermore, 0LED devices are primarily formed through deposition, and there is a pressing need for materials that can withstand long-term deposition, i.e., materials with strong heat resistance.

That is, in order to fully demonstrate the excellent characteristics of organic electronic elements, the materials forming the organic layers within the devices, such as hole injection materials, hole transport materials, emmitting materials, electron transport materials, electron injection materials, and emitting-auxiliary layer materials, must first be supported by stable and efficient materials. However, the development of stable and efficient organic layer materials for organic electronic elements has not yet been sufficiently accomplished. Therefore, the development of new materials continues to be required, and in particular, the development of materials for emitting-auxiliary layers is urgently required.

### DETAILED DESCRIPTION OF THE INVENTION

### [Summary]

In order to solve the problems of the above-described background technology, the present invention has discovered a compound having a novel structure, and has also discovered that when this compound is applied to an organic electronic element, the luminous efficiency, stability, and lifespan of the element can be greatly improved.

Accordingly, the present invention aims to provide a novel compound, an organic electronic element using the same, and an electronic device thereof.

### Technical Solution]

The present invention provides a compound represented by Formula (1).

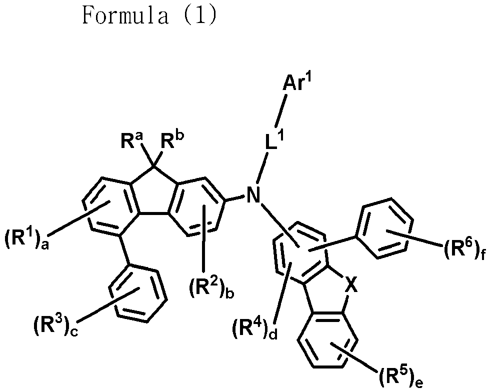

In another aspect, the present invention provides an organic electronic element comprising a compound represented by Formula (1) and an electronic device thereof.

### [Effects of the Invention]

By using the compound according to the present invention, it is possible to achieve a high luminous efficiency, a low driving voltage, and a high heat resistance of the element, and can greatly improve the color purity and lifespan of the element.

### [BRIEF DESCRIPTION OF THE DRAWINGS]

FIG. 1 to FIG. 3 illustrate an example of an organic electronic element according to the present invention.

FIG. 4 shows a Formula according to one aspect of the present invention.

| | | | |
|---|---|---|---|
| 100, 200, 300: | organic electronic element | 110 : | the first electrode |
| 120 : | hole injection layer | 130 : | hole transport layer |
| 140 : | emitting layer | 150 : | electron transport layer |
| 160 : | electron injection layer | 170 : | second electrode |
| 180 : | light efficiency enhancing Layer | 210 : | buffer layer |
| 220 : | emitting auxiliary layer | 320 : | first hole injection layer |
| 330 : | first hole transport layer | 340 : | first emitting layer |
| 350 : | first electron transport layer | 360 : | first charge generation layer |
| 361 : | second charge generation layer | 420 : | second hole injection layer |
| 430 : | second hole transport layer | 440 : | second emitting layer |
| 450 : | second electron transport layer | CGL : | charge generation layer |
| ST1 : | first stack | ST2 : | second stack |

### [DETAILED DESCRIPTION]

Hereinafter, some embodiments of the present invention will be described in detail. Further, in the following description of the present invention, a detailed description of known functions and configurations incorporated herein will be omitted when it may make the subject matter of the present invention rather unclear.

In addition, terms, such as first, second, A, B, (a), (b) or the like may be used herein when describing components of the present invention. Each of these terminologies is not used to define an essence, order or sequence of a corresponding component but used merely to distinguish the corresponding component from other component(s). It should be noted that if a component is described as being "connected", "coupled", or "connected" to another component, the component may be directly connected or connected to the other component, but another component may be "connected ", " coupled" or "connected" between each component.

As used in the specification and the accompanying claims, unless otherwise stated, the following is the meaning of the term as follows.

Unless otherwise stated, the term "halo" or "halogen" , as used herein, includes fluorine(F), bromine(Br), chlorine(Cl), or iodine(I).

Unless otherwise stated, the term "alkyl" or "alkyl group" , as used herein, has a single bond of 1 to 60 carbon atoms, 1 to 30 carbon atoms, 1 to 25 carbon atoms, 1 to 18 carbon atoms, or 1 to 12 carbon atoms, and means saturated aliphatic functional radicals including a linear alkyl group, a branched chain alkyl group, a cycloalkyl group (alicyclic), an cycloalkyl group substituted with a alkyl or an alkyl group substituted with a cycloalkyl.

Unless otherwise stated, the term "alkenyl" or "alkynyl" , as used herein, has double or triple bonds of 2 to 60 carbon atoms, 2 to 30 carbon atoms, 2 to 25 carbon atoms, 2 to 18 carbon atoms, 2 to 12 carbon atoms, but is not limited thereto, and includes a linear or a branched chain group.

Unless otherwise stated, the term "cycloalkyl" , as used herein, means alkyl forming a ring having 3 to 60 carbon atoms, 3 to 30 carbon atoms, 3 to 25 carbon atoms, 3 to 18 carbon atoms, 3 to 12 carbon atoms, but is not limited thereto.

Unless otherwise stated, the term "alkoxyl group" , "alkoxy group" or "alkyloxy group" , as used herein, means an alkyl group bonded to oxygen radical, but is not limited thereto, and has 1 to 60 carbon atoms, 1 to 30 carbon atoms, 1 to 25 carbon atoms, 1 to 18 carbon atoms, or 1 to 12 carbon atoms.

Unless otherwise stated, the term "aryloxyl group" or "aryloxy group" , as used herein, means an aryl group bonded to oxygen radical, but is not limited thereto, and has 6 to 60 carbon atoms, 6 to 30 carbon atoms, 6 to 25 carbon atoms, 6 to 18 carbon atoms, or 6 to 12 carbon atoms.

Unless otherwise specified, the terms "aryl group" and "arylene group" used in the present invention have 6 to 60 carbon atoms, 6 to 30 carbon atoms, 6 to 25 carbon atoms, 6 to 18 carbon atoms, or 6 to 12 carbon atoms, respectively, but are not limited thereto. In the present invention, an aryl group or arylene group refers to an aromatic group of a single ring or multiple rings, and comprises an aromatic ring formed by the bonding or reaction of adjacent substituents. For example, the aryl group may be phenyl, biphenyl, a fluorene group, a spirofluorene group.

The prefix "aryl" or "ar" means a radical substituted with an aryl group. For example, an arylalkyl may be an alkyl substituted with an aryl, and an arylalkenyl may be an alkenyl substituted with aryl, and a radical substituted with an aryl has a number of carbon atoms as defined herein.

Also, when prefixes are named subsequently, it means that substituents are listed in the order described first. For example, an arylalkoxy means an alkoxy substituted with an aryl, an alkoxylcarbonyl means a carbonyl substituted with an alkoxyl, and an arylcarbonylalkenyl also means an alkenyl substituted with an arylcarbonyl, wherein the arylcarbonyl may be a carbonyl substituted with an aryl.

Unless otherwise stated, the term "heterocyclic group" , as used herein, contains one or more heteroatoms, and has 2 to 60 carbon atoms, 2 to 30 carbon atoms, 2 to 25 carbon atoms, 2 to 18 carbon atoms, 2 to 12 carbon atoms, and comprises any one of a single ring or multiple ring, and may include heteroaliphatic ring and heteroaromatic ring. Also, the heterocyclic group may also be formed in conjunction with an adjacent group.

Unless otherwise stated, the term "heteroatom" , as used herein, represents at least one of N, 0, S, P, or Si.

Additionally, "heterocyclic group" means a single ring, ring aggregate, fused multiple ring system, spiro compound, etc. containing a heteroatom. Also, compounds containing heteroatom groups such as SO₂, P=0, etc. instead of carbon forming a ring, such as the compounds below, can also be included in the heterocyclic group. For example, a "heterocyclic group" includes the following compound.

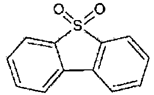

The term "aliphatic ring group" used in the present invention refers to cyclic hydrocarbons excluding aromatic hydrocarbons, and includes single rings, ring aggregates, fused multiple ring systems, spiro compounds, etc., and means a ring having 3 to 60 carbon atoms, 3 to 30 carbon atoms, 3 to 25 carbon atoms, 3 to 18 carbon atoms, 3 to 12 carbon atoms, but is not limited thereto. For example, even when benzene, an aromatic ring, and cyclohexane, a non-aromatic ring, are fused, it is an aliphatic ring.

Unless otherwise stated, the term "fluorenyl group" , "fluorenylene group" or "fluorentriyl group" as used herein, means a monovalent, divalent or trivalent functional group, in which R, R' and R" are all hydrogen in the following structures, and the term "substituted fluorenyl group" , "substituted fluorenylene group" or "substituted fluorentriyl group" means that at least one of the substituents R, R' and R" is a substituent other than hydrogen, and include those in which R and R' are bonded to each other to form a spiro compound together with the carbon to which they are bonded. In this specification, fluorenyl group, fluorenylene group, and fluorenetriyl group may all be referred to as fluorene groups, regardless of valence.

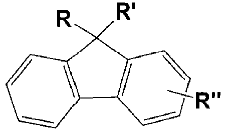

The term "spiro compound" used in the present invention has a 'spiro union', and a spiro union means a connection formed by 2 rings sharing only one atom. At this time, the atom shared between the 2 rings is called a 'spiro atom', and depending on the number of spiro atoms contained in a compound, they are called 'monospiro-', 'dispiro-', and 'trispiro-' compounds, respectively.

Unless otherwise stated, the term "aliphatic" as used herein means an aliphatic hydrocarbon having 1 to 60 carbon atoms, 1 to 30 carbon atoms, 1 to 25 carbon atoms, 1 to 18 carbon atoms or 1 to 12 carbon atoms, and "aliphatic ring" means an aliphatic hydrocarbon ring having 3 to 60 carbon atoms, 3 to 30 carbon atoms, 3 to 25 carbon atoms, 3 to 18 carbon atoms or 3 to 12 carbon atoms.

Unless otherwise stated, the term "ring" , as used herein, means an aliphatic ring having 3 to 60 carbon atoms, 3 to 30 carbon atoms, 3 to 25 carbon atoms, 3 to 18 carbon atoms or 3 to 12 carbon atoms; or an aromatic ring having 6 to 60 carbon atoms, 6 to 30 carbon atoms, 6 to 25 carbon atoms, 6 to 18 carbon atoms, or 6 to 12 carbon atoms; or a heterocyclic having 2 to 60 carbon atoms, 2 to 30 carbon atoms, 2 to 25 carbon atoms, 2 to 18 carbon atoms, 2 to 12 carbon atoms, or a fused ring formed by the combination thereof, and includes a saturated or unsaturated ring.

Other hetero compounds or hetero radicals other than the above-mentioned hetero compounds include, but are not limited thereto, one or more heteroatoms.

Also, unless expressly stated, as used herein, "substituted" in the term "substituted or unsubstituted" means substituted with one or more substituents selected from the group consisting of deuterium, halogen, an amino group, a nitrile group, a nitro group, a C₁-C₂₀ alkyl group, a C₁-C₂₀ alkoxyl group, a C₁-C₂₀ alkylamine group, a C₁-C₂₀ alkylthiopen group, a C₆-C₂₀ arylthiopen group, a C₂-C₂₀ alkenyl group, a C₂-C₂₀ alkynyl group, a C₃-C₂₀ cycloalkyl group, a C₆-C₂₀ aryl group, a C₆-C₂₀ aryl group substituted by deuterium, a C₈-C₂₀ arylalkenyl group, a silane group, a boron group, a germanium group, and a C₂-C₂₀ heterocyclic group, but is not limited to these substituents.

In this specification, the 'group name' corresponding to the aryl group, arylene group, heterocyclic group, etc., as examples of each symbol and its substituent, may be written as the 'name of the group reflecting the valence', but is written as the 'parent compound name'. For example, in the case of 'phenanthrene', a type of aryl group, the name of the group may be written by distinguishing the valence, such as the monovalent 'group' is 'phenanthryl' and the divalent group is 'phenanthrylene', but may be written as 'phenanthrene', which is the name of the parent compound, regardless of the valence. Similarly, in the case of pyrimidine, it can be written as 'pyrimidine' regardless of the valence, or it can be written as the 'name of the group' of the valence, such as pyrimidineyl group in the case of monovalent group, pyrimidineylene in the case of divalent group, etc.

Additionally, in this specification, when describing compound names or substituent names, numbers or alphabets indicating positions may be omitted. For example, pyrido[4,3-d]pyrimidine to pyridopyrimidine, benzofuro[2,3-d]pyrimidine to benzofuropyrimidine, 9,9-dimethyl-9H-fluorene can be described as dimethylfluorene, etc. Therefore, both benzo[g]quinoxaline and benzo[f]quinoxaline can be described as benzoquinoxaline.

Also, unless there is an explicit explanation, the formula used in the present invention is the same as the definition of the substituent by the exponent definition of the following formula.

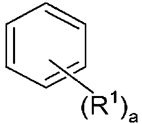

Here, when a is an integer of 0, the substituent R¹ is absent, when a is an integer of 1, the sole substituent R¹ is linked to any one of the carbon constituting the benzene ring, when a is an integer of 2 or 3, each is combined as follows, where R¹ may be the same or different from each other, when a is an integer of 4 to 6, it is bonded to the carbon of the benzene ring in a similar manner, while the indication of the hydrogen bonded to the carbon forming the benzene ring is omitted.

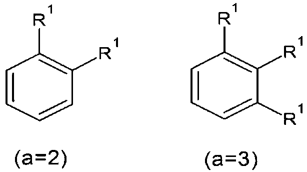

Unless otherwise expressly stated, the terms "ortho", "meta", and "para" used in the present invention refer to the substitution positions of all substituents, and the ortho position refers to a compound in which the position of the substituent is immediately adjacent, for example, when benzene is used, it means 1 or 2 position, and the meta position is the next substitution position of the neighbor substitution position, when benzene as an example stands for 1 or 3 position, and the para position is the next substitution position of the meta position, which means 1 and 4 position when benzene is taken as an example. A more detailed example of the substitution position is as follows, and it can be confirmed that the ortho-, and meta- position are substituted by non-linear type and para- positions are substituted by linear type.

### [Example of or tho-position]

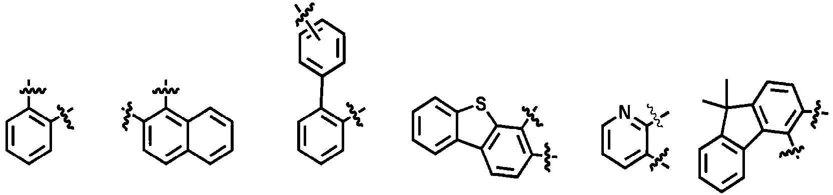

### [Example of meta-position]

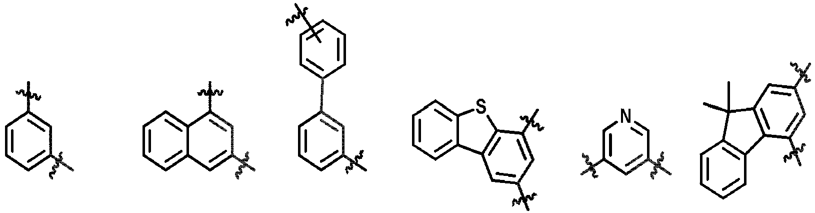

### [Example of para-position]

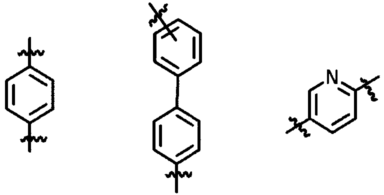

Hereinafter, a compound according to one aspect of the present invention and an organic electronic element comprising the same will be described.

The present invention provides a compound represented by Formula (1). 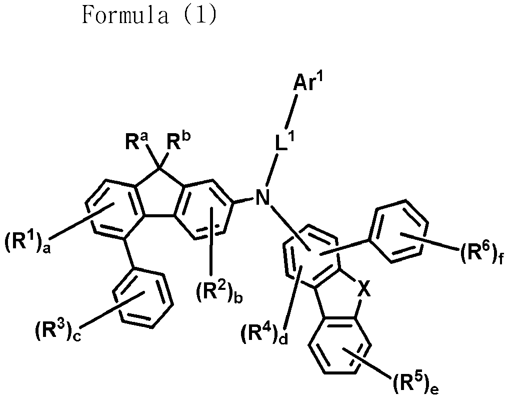 wherein:
1) X is 0 or S,
2) Ar¹ is selected from the group consisting of a C₆-C₆₀ aryl group; a fluorenyl group; a C₂-C₆₀ heterocyclic group including at least one heteroatom of 0, N, S, Si and P; and a C₃-C₆₀ aliphatic ring;
   wherein when Ar¹ is a aryl group, preferably a C₆-C₃₀ aryl group, more preferably a C₆-C₂₅ aryl group, an a C₆-C₁₈ aryl group or an a C₆-C₁₂ aryl group, such as phenyl, biphenyl, terphenyl, naphthalene, phenanthrene, chyrisen, etc.
   wherein when Ar¹ is a heterocyclic group, preferably a C₂-C₃₀ heterocyclic group, more preferably a C₂-C₂₅ heterocyclic group, a C₂-C₁₈ heterocyclic group, or a C₂-C₁₂ heterocyclic group, such as pyrazine, thiophene, pyridine, pyrimidine, quinoline, pyrimidoindole, 5-phenyl-5H-pyrimido[5,4-b]indole, quinazoline, quinoxaline, benzoquinazoline, carbazole, dibenzoquinazoline, benzofuran, benzothiophene, dibenzofuran, dibenzothiophene, benzothienopyrimidine, benzofuropyrimidine, phenothiazine, phenylphenothiazine, naphthobenzofuran, naphthobenzothiophene, etc.
   wherein when Ar¹ is an aliphatic ring group, preferably a C₃-C₃₀ aliphatic ring group, more preferably a C₃-C₂₅ aliphatic ring group, a C₃-C₁₈ aliphatic ring group, and a C₃-C₁₂ aliphatic ring group, and specifically, cyclobutane, cyclopentane, cyclohexane, bicycloheptane, adamantyl, etc.
3) L¹ is independently a single bond; a C₆-C₆₀ arylene group; or a C₂-C₆₀ heterocyclic group;
   wherein when L¹ is a arylene group, preferably a C₆-C₃₀ arylene group, more preferably a C₆-C₂₅ arylene group, a C₆-C₁₈ arylene group or a C₆-C₁₂ arylene group, such as phenylene, biphenylene, naphthylene, terphenylene, anthracenylene, phenanthrenylene, etc.
   wherein when L¹ is a heterocyclic group, preferably a C₂-C₃₀ heterocyclic group, more preferably a C₂-C₂₅ heterocyclic group, a C₂-C₁₈ heterocyclic group, or a C₂-C₁₂ heterocyclic group, such as pyrazine, thiophene, pyridine, pyrimidine, quinoline, pyrimidoindole, 5-phenyl-5H-pyrimido[5,4-b]indole, quinazoline, quinoxaline, benzoquinazoline, carbazole, dibenzoquinazoline, benzofuran, benzothiophene, dibenzofuran, dibenzothiophene, benzothienopyrimidine, benzofuropyrimidine, phenothiazine, phenylphenothiazine, naphthobenzofuran, naphthobenzothiophene, etc.
4) R¹, R², R³, R⁴, R⁵ and R⁶ are independently the same or different from each other and are independently selected from the group consisting of hydrogen; deuterium; halogen; a C₁-C₅₀ alkyl group; a C₂-C₂₀ alkenyl group; a C₂-C₂₀ alkynyl group; a C₁-C₃₀ alkoxy group; and a C₆-C₃₀ a aryloxy group; a C₆-C₆₀ aryl group; and a C₂-C₆₀ heterocyclic group;
   wherein when R¹, R², R³, R⁴, R⁵ and R⁶ are a alkyl group, preferably a C₁-C₃₀ alkyl group, more preferably a C₁-C₂₅ alkyl group, a C₁-C₁₈ alkyl group or a C₁-C₁₂ alkyl group, such as a methyl group, ethyl group, propyl group, isopropyl group, butyl group, t-butyl group, pentyl group, etc.
   wherein when R¹, R², R³, R⁴, R⁵ and R⁶ are a alkoxyl group, preferably a C₁-C₂₅ alkoxyl group, more preferably a C₁-C₁₈ alkoxyl group, or a C₁-C₁₂ alkoxyl group,
   wherein when R¹, R², R³, R⁴, R⁵ and R⁶ are a aryloxy group, preferably a C₆ C₂₅ aryloxy group, more preferably a C₆-C₁₈ aryloxy group, or a C₆-C₁₂ aryloxy group.
   wherein when R¹, R², R³, R⁴, R⁵ and R⁶ are a aryl group, preferably a C₆-C₃₀ aryl group, more preferably a C₆-C₂₅ aryl group, a C₆-C₁₈ aryl group or a C₆-C₁₂ aryl group, such as phenyl, biphenyl, terphenyl, naphthalene, phenanthrene, chrysene, etc.
   wherein when R¹, R², R³, R⁴, R⁵ and R⁶ are a heterocyclic group, preferably a C₂-C₃₀ heterocyclic group, more preferably a C₂-C₂₅ heterocyclic group, a C₂-C₁₈ heterocyclic group, or a C₂-C₁₂ heterocyclic group, such as pyrazine, thiophene, pyridine, pyrimidine, quinoline, pyrimidoindole, 5-phenyl-5H-pyrimido[5,4-b]indole, quinazoline, quinoxaline, benzoquinazoline, carbazole, dibenzoquinazoline, benzofuran, benzothiophene, dibenzofuran, dibenzothiophene, benzothienopyrimidine, benzofuropyrimidine, phenothiazine, phenylphenothiazine, naphthobenzofuran, naphthobenzothiophene, etc.
5) R^{a} and R^{b} are independently a C₁-C₅₀ alkyl group; or a C₆-C₆₀ aryl group,
   Wherein when R^{a} and R^{b} are a alkyl group, preferably a C₁-C₃₀ alkyl group, more preferably a C₁-C₂₅ alkyl group, a C₁-C₁₈ alkyl group or a C₁-C₁₂ alkyl group, such as a methyl group, ethyl group, propyl group, isopropyl group, butyl group, t-butyl group, pentyl group, etc.
   Wherein when R^{a} and R^{b} are a aryl group, preferably a C₆-C₃₀ aryl group, more preferably a C₆-C₂₅ aryl group, a C₆-C₁₈ aryl group or a C₆-C₁₂ aryl group, such as phenyl, biphenyl, terphenyl, naphthalene, phenanthrene, chrysene, etc.
6) a and b are independently an integer of 0 to 3, d is an integer of 0 to 2, e is an integer of 0 to 4, c and f are independently an integer of 0 to 5,
wherein the aryl group, arylene group, heterocyclic group, fluorenyl group, aliphatic ring group, alkyl group, alkenyl group, alkynyl group, alkoxyl group and aryloxy group may be substituted with one or more substituents selected from the group consisting of deuterium; halogen; silane group; siloxane group; boron group; germanium group; a cyano group; a nitro group; a C₁-C₂₀ alkylthio group; a C₁-C₂₀ alkoxyl group; a C₆-C₂₀ aryloxy group; a C₁-C₂₀ alkyl group; a C₂-C₂₀ alkenyl group; a C₂-C₂₀ alkynyl group; a C₆-C₂₀ aryl group; a C₆-C₂₀ aryl group substituted with deuterium; a fluorenyl group; a C₂-C₂₀ heterocyclic group; a C₃-C₂₀ aliphatic ring; a C₇-C₂₀ arylalkyl group; a C₈-C₂₀ arylalkenyl group; and a C₇-C₂₀ alkylaryl group; and the hydrogen of these substituents may be further substituted with one or more deuterium, and the substituents may be bonded to each other to form a saturated or unsaturated ring, wherein the term 'ring' means a C₃-C₆₀ aliphatic ring or a C₆-C₆₀ aromatic ring or a C₂-C₆₀ heterocyclic group or a fused ring formed by the combination thereof.

Formula (1) is represented by any one of Formulas (2) to (5). 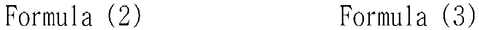 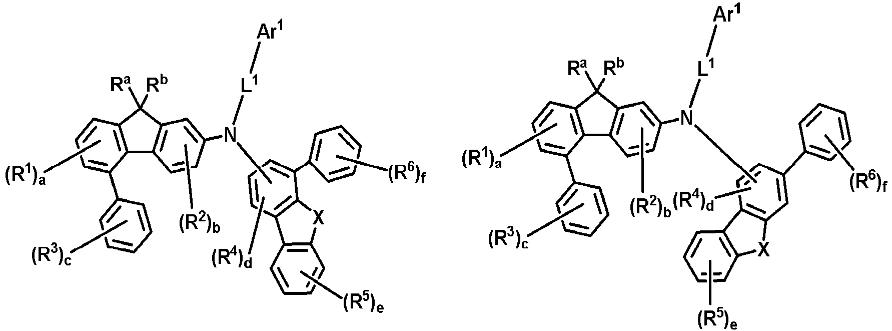 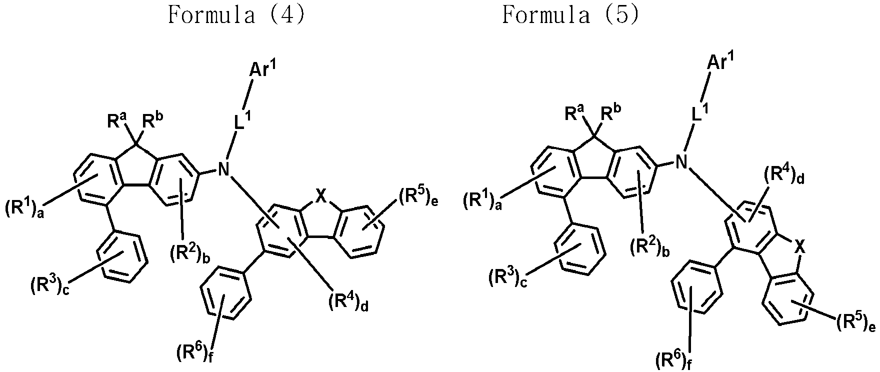 wherein, X, Ar¹, L¹, R¹, R², R³, R⁴, R⁵, R⁶, R^{a}, R^{b}, a, b, c, d, e and f are the same as defined in Formula (1)

Formula (1) is represented by any one of Formulas (6) to (9). 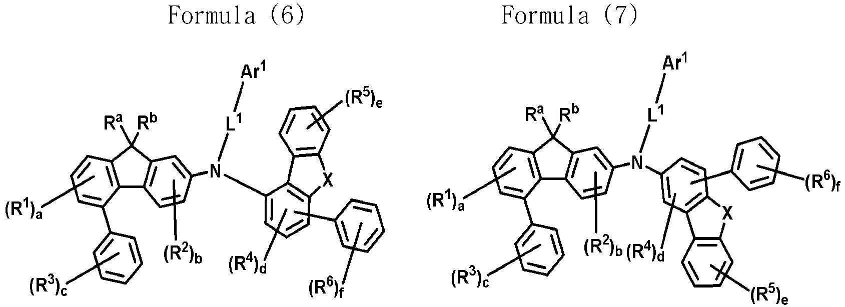 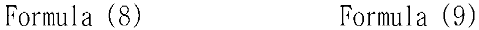 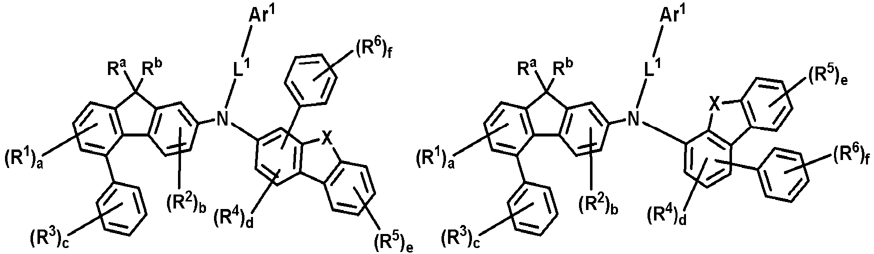 wherein, X, Ar¹, L¹, R¹, R², R³, R⁴, R⁵, R⁶, R^{a}, R^{b}, a, b, c, d, e and f are the same as defined in Formula (1).

Formula (1) is represented by any one of Formulas (10) to (15). 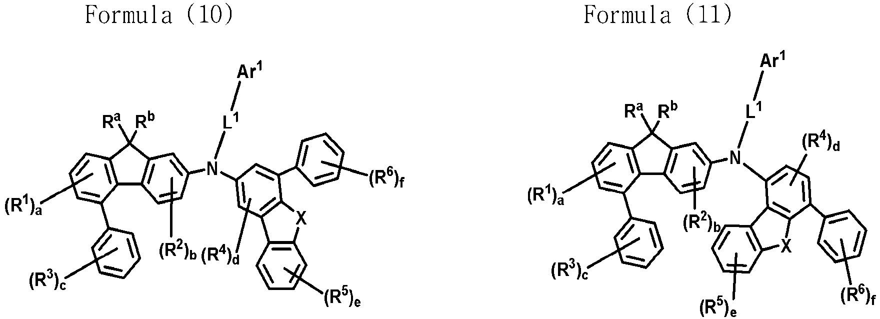 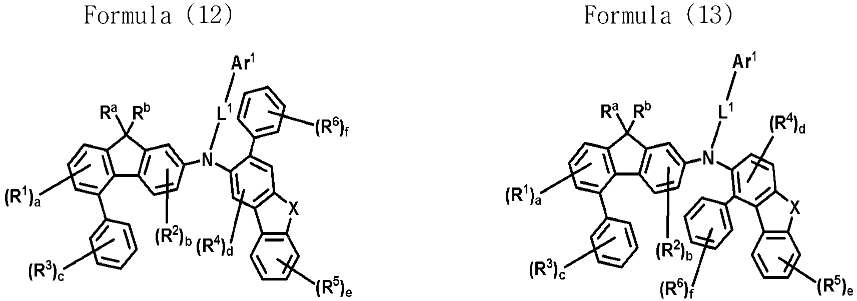 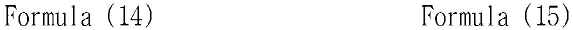 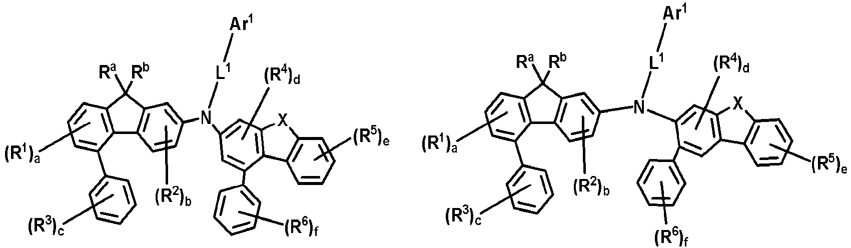 wherein, X, Ar¹, L¹, R¹, R², R³, R⁴, R⁵, R⁶, R^{a}, R^{b}, a, b, c, d, e and f are the same as defined in Formula (1).

Also, preferably the Ar¹ is a C₆-C₃₀ aryl group; a fluorenyl group; or a C₆-C₃₀ heterocyclic group.

More preferably Ar¹ is represented by a C₆-C₁₈ aryl group; or one of the Formula (Ar-1) to Formula (Ar-4). 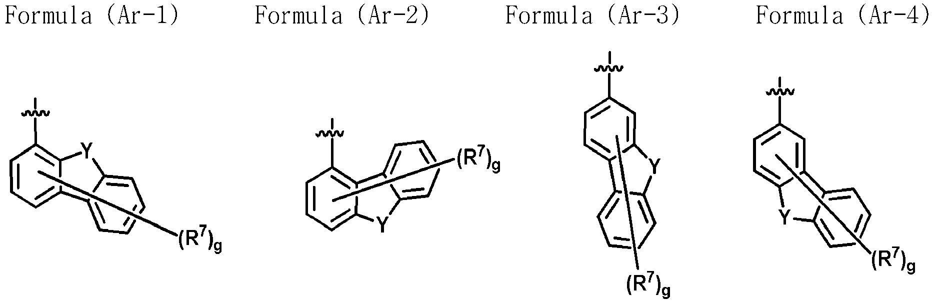 wherein:
Y is 0, S or CR'R",
R⁷ is independently the same or different from each other and is independently selected from the group consisting of hydrogen; deuterium; halogen; silane group; siloxane group; boron group; germanium group; cyano group; nitro group; a C₁-C₂₀ alkylthio group; a C₁-C₂₀ alkoxy group; a C₁-C₂₀ alkyl group; a C₂-C₂₀ alkenyl group; a C₂-C₂₀ alkynyl group; a C₆-C₂₀ aryl group; a C₆-C₂₀ aryl group substituted with deuterium; a fluorenyl group; a C₂-C₂₀ heterocyclic group; a C₃-C₂₀ cycloalkyl ring; a C₇-C₂₀ arylalkyl group; and a C₈-C₂₀ arylalkenyl group; or a plurality of adjacent groups thereof may be bonded to each other to form a ring,
R' and R" are independently a C₆-C₆₀ aryl group substituted or unsubstituted with deuterium; or a C₁-C₅₀ alkyl group substituted or unsubstituted with deuterium;
Wherein when R' and R" are an aryl group, preferably a C₆-C₃₀ aryl group, more preferably a C₆-C₂₅ aryl group, a C₆-C₁₈ aryl group or a C₆-C₁₂ aryl group, such as phenyl, biphenyl, terphenyl, naphthalene, phenanthrene, chrysene, etc.
Wherein when R' and R" are an alkyl group, preferably a C₁-C₃₀ alkyl group, more preferably a C₁-C₂₅ alkyl group, a C₁-C₁₈ alkyl group or a C₁-C₁₂ alkyl group, such as a methyl group, ethyl group, propyl group, isopropyl group, butyl group, t-butyl group, pentyl group, etc.
g is an integer of 0 to 7, 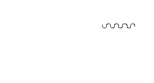 indicates the position to be bonded.

Specifically, the compound represented by Formula (1) may be represented by any one of the following compounds P-1 to P-120, but is not limited thereto.

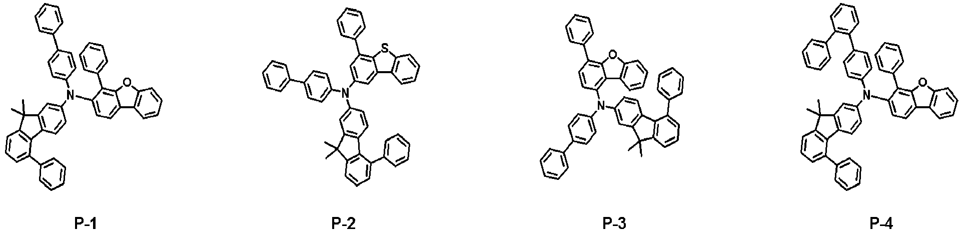

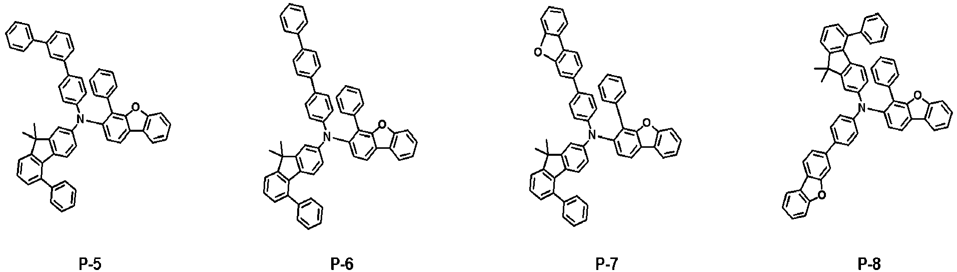

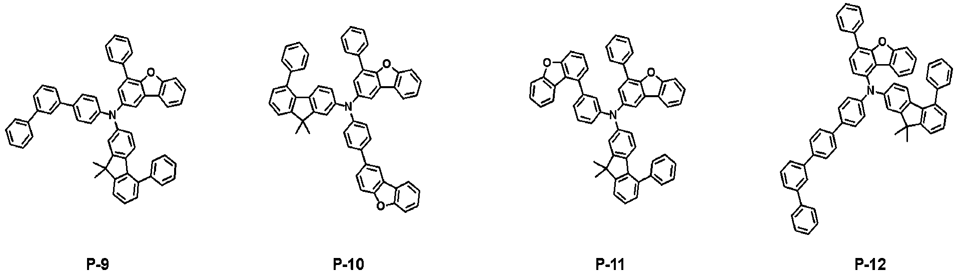

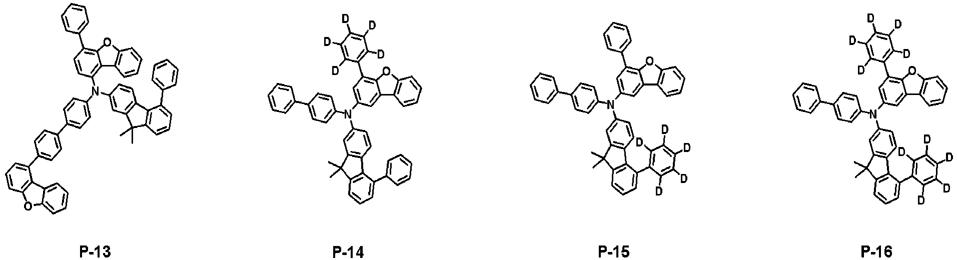

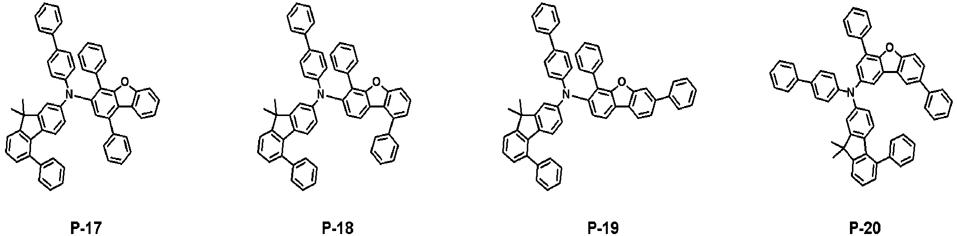

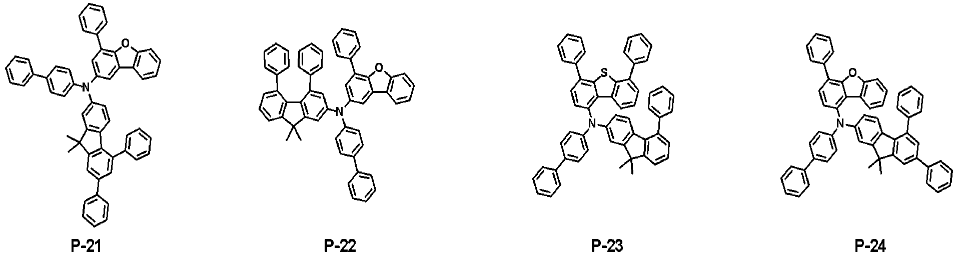

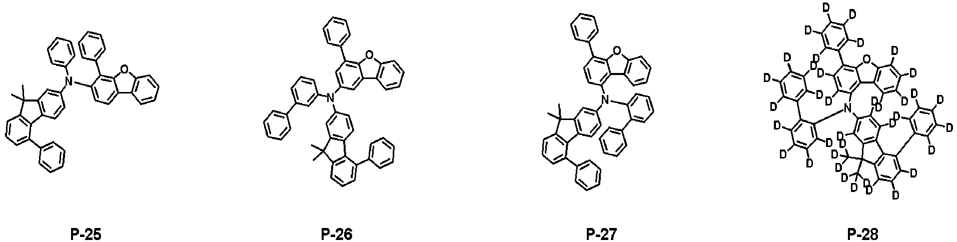

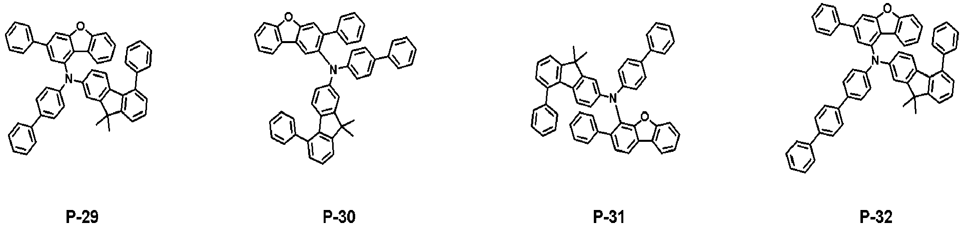

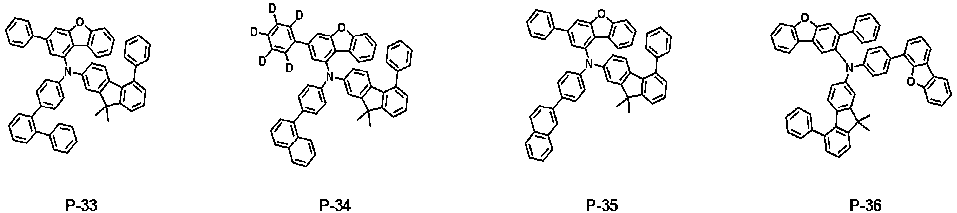

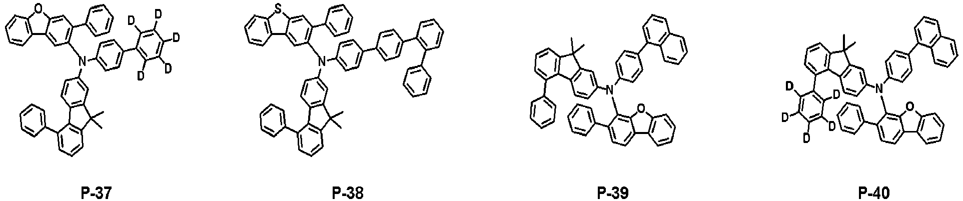

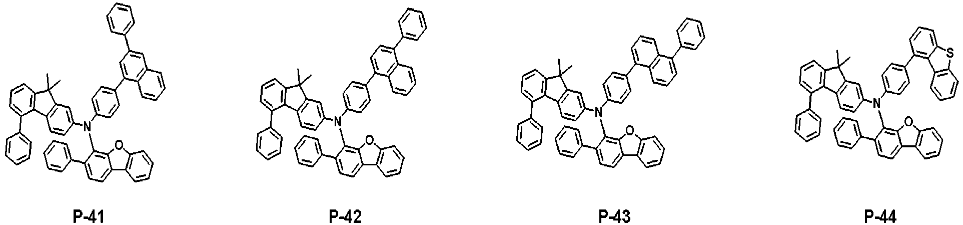

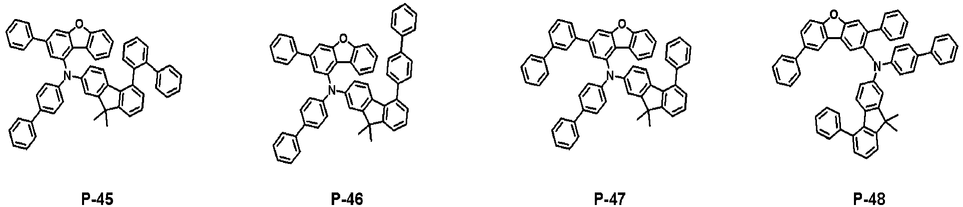

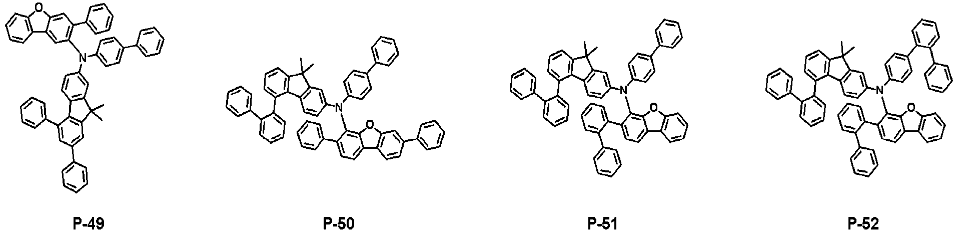

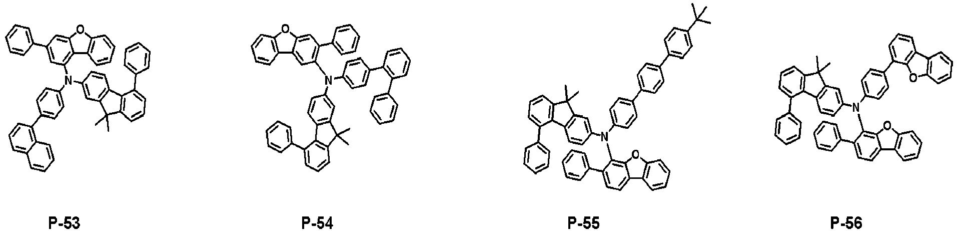

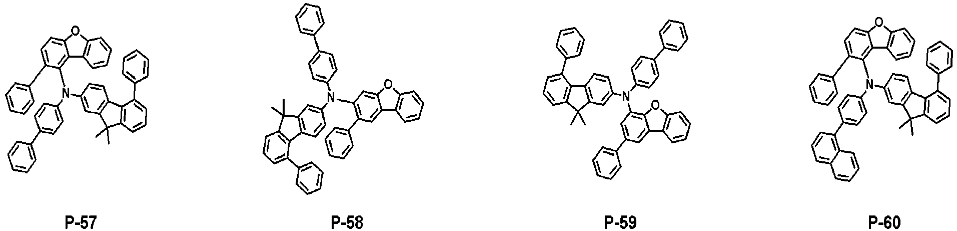

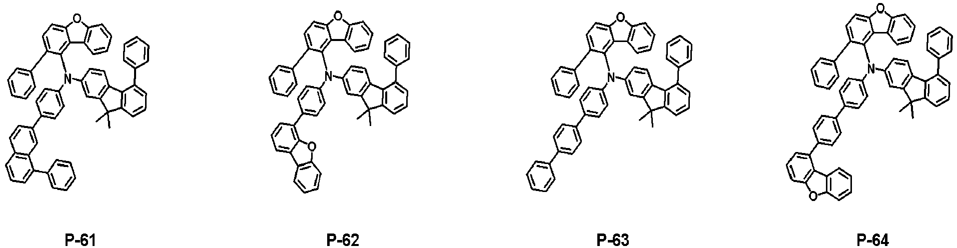

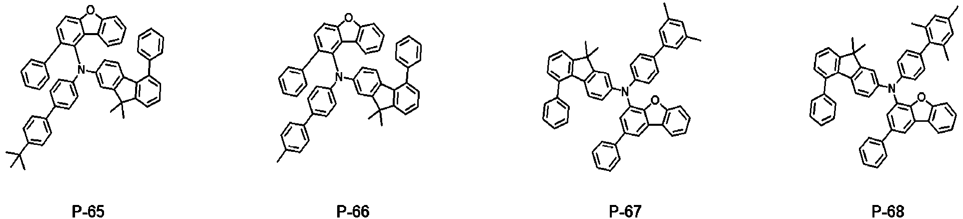

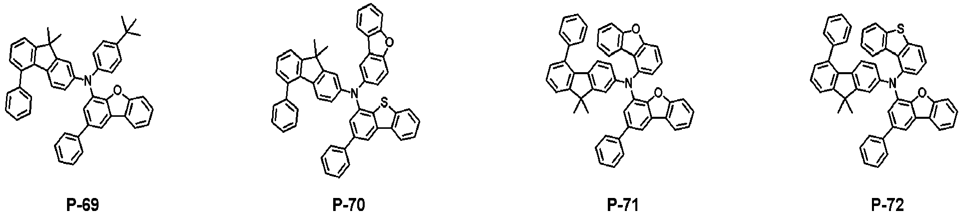

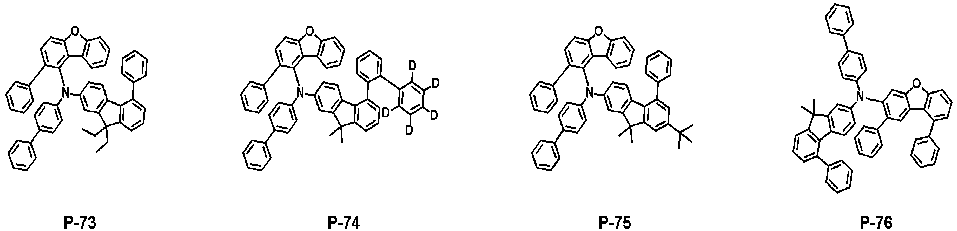

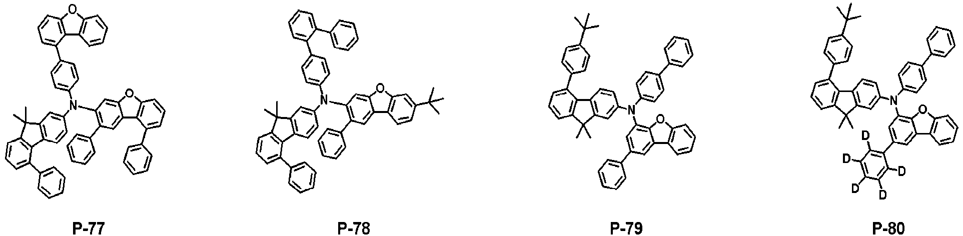

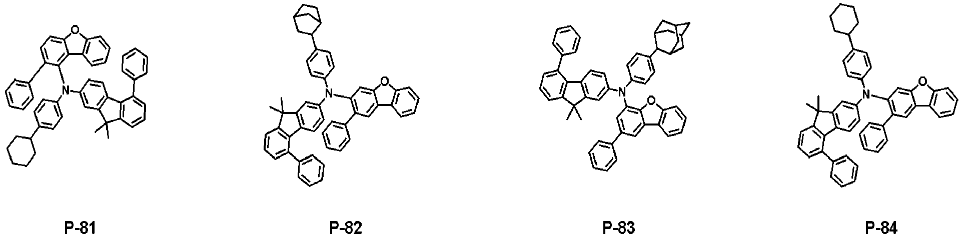

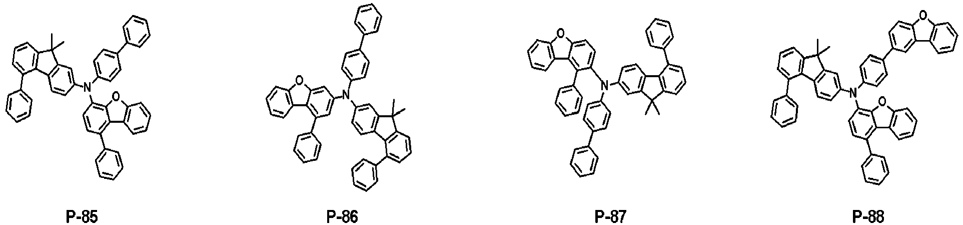

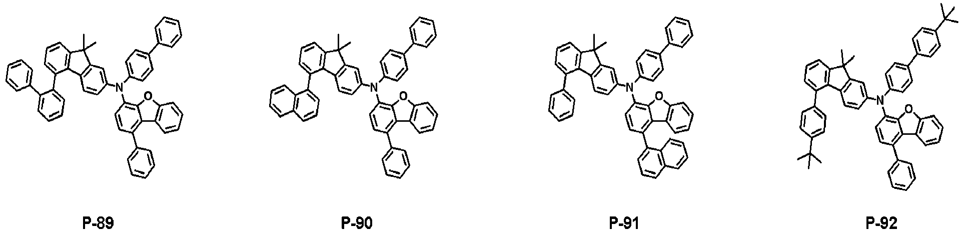

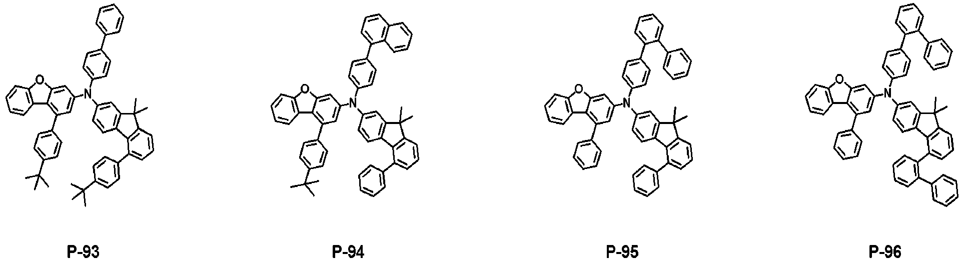

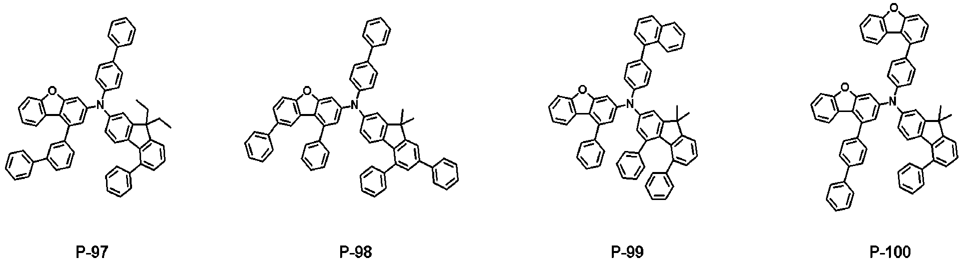

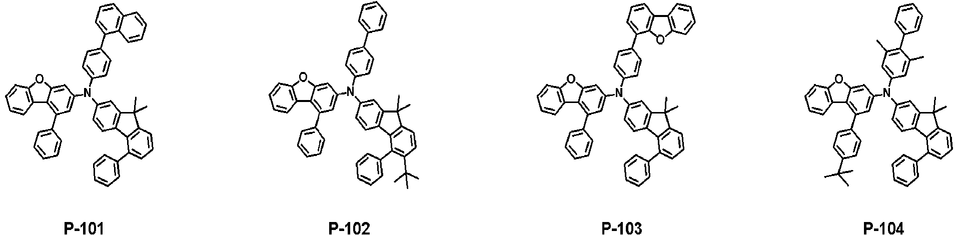

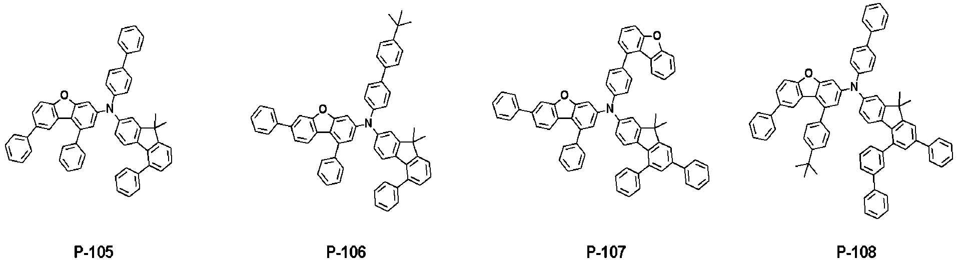

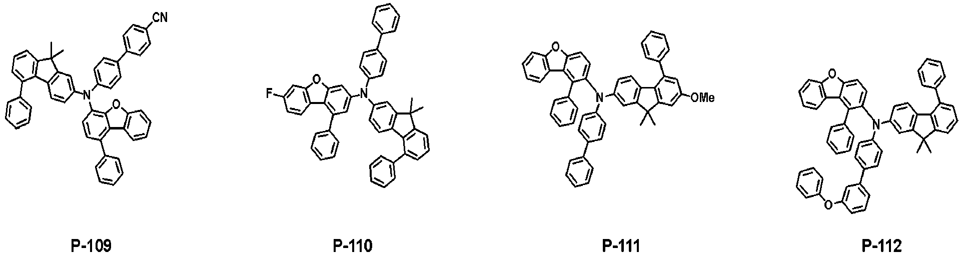

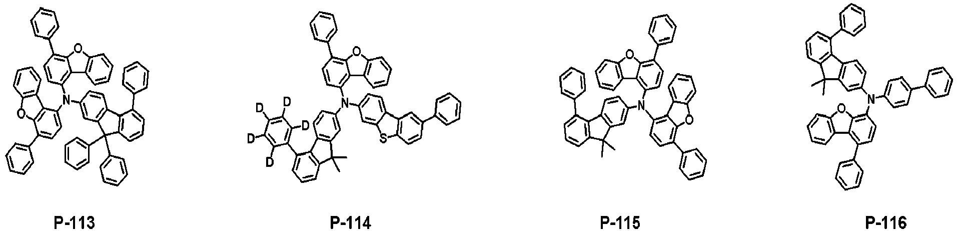

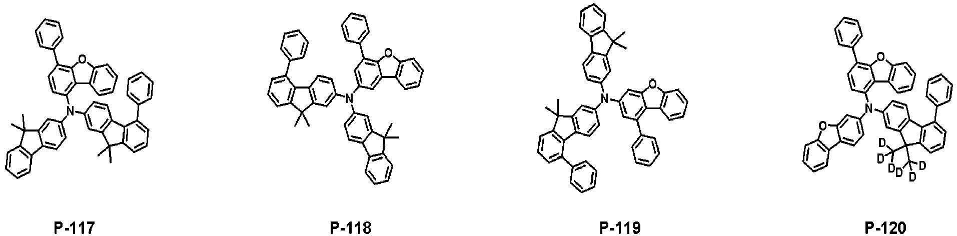

In another aspect, the present invention provides a method for reusing a compound represented by Formula (1) comprising:
recovering a crude organic light emitting material comprising the compound represented by Formula (1) from a deposition apparatus used in the process for depositing the organic emitting material to prepare an organic light emitting device;
removing impurities from the crude organic light emitting material;
recovering the organic light emitting material after the impurities are removed; and
purifying the recovered organic light emitting material to have a purity of 99.9% or higher.

The step of removing impurities from the crude organic light emitting material recovered from the deposition apparatus may preferably comprise performing a pre-purification process to obtain a purity of 98% or more by recrystallization in a recrystallization solvent.

The recrystallization solvent may be preferably a polar solvent having a polarity index (PI) of 5.5 to 7.2.

The recrystallization solvent may be preferably a polar solvent having a polarity index (PI) of 5.5 to 7.2.

The recrystallization solvent may preferably be used by mixing a polar solvent having a polarity value of 5.5 to 7.2 and a non-polar solvent having a polarity value of 2.0 to 4.7.

When a mixture of a polar solvent and a non-polar solvent is used, the recrystallization solvent may be used in an amount of 15% (v/v) or less of the non-polar solvent compared to the polar solvent.

The recrystallization solvent is preferably a single solvent of N-Methylpyrrolidone (NMP); or a polar solvent mixed any one selected from the group consisting of 1,3-Dimethyl-2-imidazolidinone, 2-pyrrolidone, N,N-Dimethyl formamide, Dimethyl acetamide, and Dimethyl sulfoxide to the N-Methylpyrrolidone; or alone; or mixed non-polar solvents; selected from the group consisting of Toluene, Dichloromethane (DCM), Dichloroethane (DCE), Tetrahydrofuran (THF), Chloroform, Ethyl acetate and Butanone; or a mixture of a polar solvent and a non-polar solvent.

The pre-purification process may comprise a step of precipitating crystals of by cooling to 0° C. to 5° C. after dissolving the crude organic light emitting material recovered from the deposition apparatus in a polar solvent at 90° C. to 120° C.

The pre-purification process may comprise a step of precipitating crystals by cooling to 35° C to 40° C, adding a non-polar solvent, and then cooling to 0° C to 5° C. after dissolving the crude organic light emitting material recovered from the deposition apparatus in a polar solvent at 90° C. to 120° C.

The pre-purification process may comprise a step of precipitating crystals while concentrating the solvent and removing the non-polar solvent, after dissolving the crude organic light emitting material recovered from the deposition apparatus in a non-polar solvent.

The pre-purification process may comprise a step of recrystallizing again with a non-polar solvent after recrystallizing first with a polar solvent.

The step of purifying the recovered impurities to a purity of 99.9% or higher may comprise performing an adsorption separation process to adsorb and remove impurities by adsorbing on the adsorbent.

The adsorbent may be activated carbon, silica gel, alumina, or a material for known adsorption purposes.

The step of purifying the recovered impurities to a purity of 99.9% or higher may comprise performing sublimation purification.

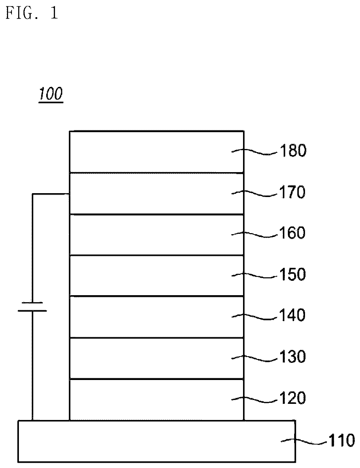

Referring to FIG. 1, an organic electronic element (100) according to the present invention comprises a first electrode (110), a second electrode (170), and an organic material layer comprising a single compound or 2 or more compounds represented by Formula (1) between the first electrode (110) and the second electrode (170). Wherein, the first electrode (110) may be an anode or positive electrode, and the second electrode (170) may be a cathode or a negative electrode. In the case of an inverted organic electronic element, the first electrode may be a cathode, and the second electrode may be an anode.

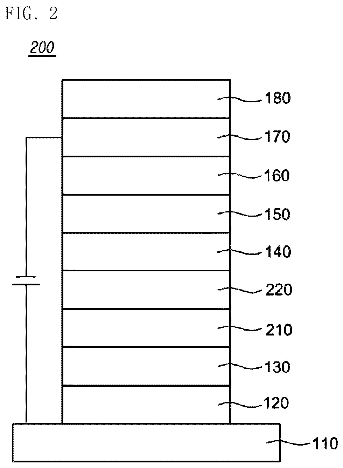

The organic material layer may sequentially comprise a hole injection layer (120), a hole transport layer (130), an emitting layer (140), an electron transport layer(150), and an electron injection layer (160) on the first electrode (110). Here, the remaining layers except the emitting layer (140) may not be formed. The organic material layer may further comprise a hole blocking layer, an electron blocking layer, an emitting-auxiliary layer (220), a buffer layer (210), etc., and the electron transport layer (150), etc. may serve as a hole blocking layer (see FIG. 2).

Additionally, the organic electronic element according to an embodiment of the present invention may further include a protective layer or a light efficiency enhancing layer (180). Wherein the light efficiency enhancing layer is formed on one of both surfaces of the first electrode that is not in contact with the organic material layer or on one of both surfaces of the second electrode that is not in contact with the organic material layer. The compound according to an embodiment of the present invention applied to the organic material layer may be used as a material for a hole injection layer (120), a hole transport layer (130), an emitting-auxiliary layer (220), an electron transport auxiliary layer, an electron transport layer (150), an electron injection layer (160), a host or dopant of an emitting layer (140), or the light efficiency enhancing layer. Preferably, for example, a compound according to Formula (1) of the present invention can be used as a material of an emitting-auxiliary layer.

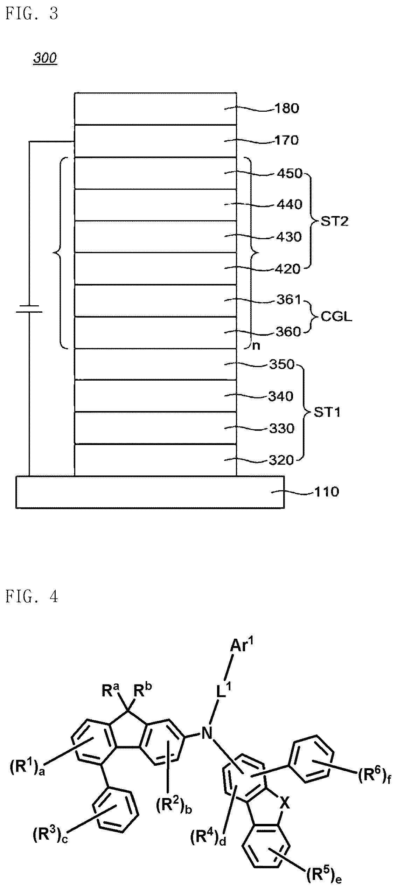

The organic material layer may comprise 2 or more stacks comprising a hole transport layer, an emitting layer and an electron transport layer sequentially formed on the anode, and may further comprise a charge generation layer formed between the 2 or more stacks (see FIG. 3).

Otherwise, even if the same core is used, the band gap, the electrical characteristics, the interface characteristics, etc. may vary depending on which substituent is bonded at which position, therefore the choice of core and the combination of sub-substituents associated therewith is also very important, and in particular, when the optimal combination of energy levels and T1 values, and unique properties of materials(mobility, interfacial characteristics, etc.) of each organic material layer is achieved, a long life span and high efficiency can be achieved at the same time.

The organic electroluminescent device according to an embodiment of the present invention may be manufactured using a PVD (physical vapor deposition) method. For example, a metal or a metal oxide having conductivity or an alloy thereof is deposited on a substrate to form a cathode, and the organic material layer including the hole injection layer(120), the hole transport layer(130), the emitting layer(140), the electron transport layer(150), and the electron injection layer(160) is formed thereon, and then a material that can be used as a cathode is deposited thereon.

Also, the present invention provides the organic electronic element wherein the organic material layer is formed by one of a spin coating process, a nozzle printing process, an inkjet printing process, a slot coating process, a dip coating process or a roll-to-roll process, and the organic material layer comprises the compound as an electron transport material.

As another specific example, a compound of the same or different types represented by Formula (1) is mixed and used in the organic material layer.

Additionally, the present invention provides an emitting-auxiliary layer composition comprising a compound represented by Formula (1), and provides an organic electronic element comprising the emitting-auxiliary layer.

Also, the present invention also provides an electronic device comprising a display device comprising the organic electronic element; and a control unit for driving the display device.

According to another aspect, the present invention provides a display device wherein the organic electronic element is at least one of an OLED, an organic solar cell, an organic photo conductor, an organic transistor (organic TFT) and an element for monochromic or white illumination. Here, the electronic device may be a wired/wireless communication terminal which is currently used or will be used in the future, and covers all kinds of electronic devices including a mobile communication terminal such as a cellular phone, a personal digital assistant(PDA), an electronic dictionary, a point-to-multipoint(PMP), a remote controller, a navigation unit, a game player, various kinds of TVs, and various kinds of computers.

Hereinafter, Synthesis examples of the compound represented by Formula (1) of the present invention, and preparation examples of the organic electronic element will be described in detail by way of example, but are not limited to the following examples.

### [Synthesis Example]

The compound (final products) represented by Formula (1) according to the present invention can be synthesized by a reaction as in Reaction Scheme 1, but is not limited thereto. (Hal= Br, I or Cl)

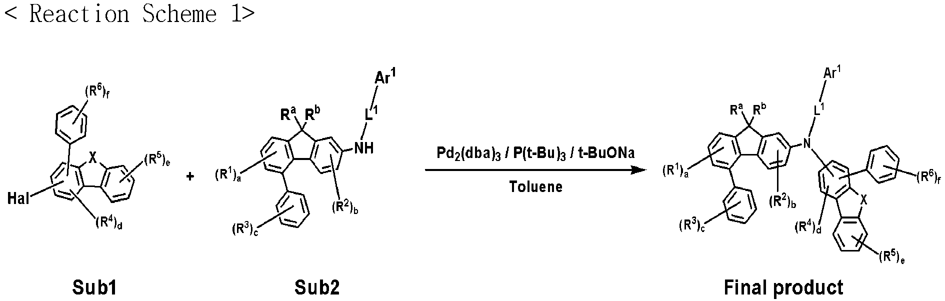

### I. Synthesis of Sub1

Sub1 of the reaction scheme 1 is synthesized by the reaction path of the reaction scheme 2, but is not limited thereto. (Hal= Br, I or Cl)

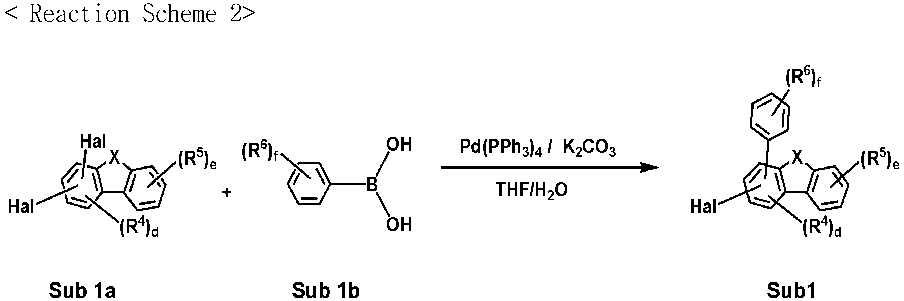

### 1. Synthesis of Sub 1-1

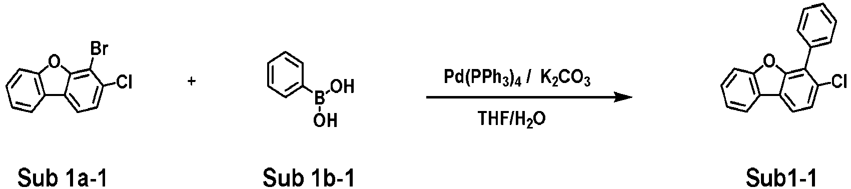

Sub 1a-1 (50 g, 177.60 mmol) and Sub 1b-1 (23.8 g, 195.36 mmol) were dissolved in THF (440mL) and water (150 ml) in a round-bottom flask, Pd(PPh₃)₄ (6.1 g, 5.32 mmol), K₂CO₃ (49.1 g, 355.20 mmol) were added and stirred at 70° C. After the reaction was completed, the mixture was extracted with Toluene and water, the organic layer was dried over MgSO₄ and concentrated, and the resulting compound was recrystallized using a silicagel column to obtain 44.0 g of Sub1-1 (yield: 88.9%).

### 2. Synthesis of Sub 1-11

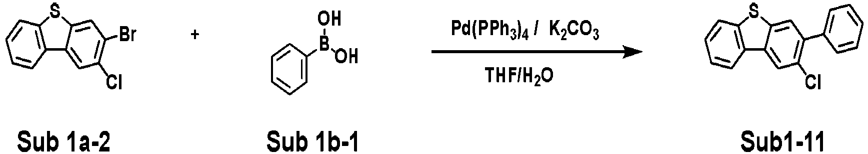

Sub 1a-2 (50 g, 168.02 mmol) and Sub 1b-1 (22.5 g, 184.82 mmol) were dissolved in THF (420mL) and water (140 ml) in a round-bottom flask, Pd(PPh₃)₄ (5.8 g, 5.04 mmol), K₂CO₃ (46.4 g, 336.03 mmol) were added and stirred at 70° C. After the reaction was completed, the mixture was extracted with Toluene and water, the organic layer was dried over MgSO₄ and concentrated, and the resulting compound was recrystallized using a silicagel column to obtain 45.0 g of Sub1-11 (yield: 90.8%).

### 3. Synthesis of Sub 1-15

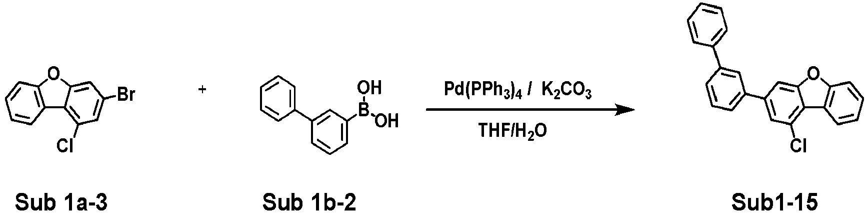

Sub 1a-3 (50 g, 177.60 mmol) and Sub 1b-2 (38.69 g, 195.36 mmol) were dissolved in THF (440mL) and water (150 ml) in a round-bottom flask, Pd(PPh₃)₄ (6.1 g, 5.33 mmol), K₂CO₃ (49.1 g, 355.20 mmol) were added and stirred at 70° C. After the reaction was completed, the mixture was extracted with Toluene and water, the organic layer was dried over MgSO₄ and concentrated, and the resulting compound was recrystallized using a silicagel column to obtain 55.0 g of Sub1-15 (yield: 87.3%).

### 4. Synthesis of Sub 1-27

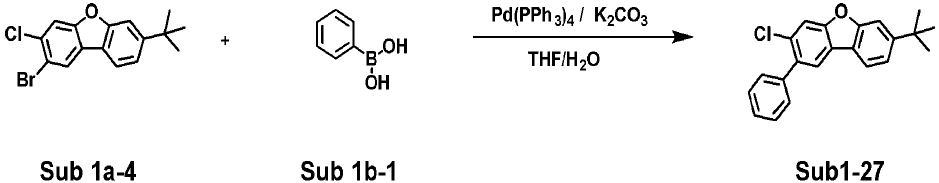

Sub 1a-4 (50 g, 148.08 mmol) and Sub 1b-1 (19.8 g, 162.89 mmol) were dissolved in THF (490mL) and water (120 ml) in a round-bottom flask, Pd(PPh₃)₄ (5.1 g, 4.44 mmol), K₂CO₃ (40.9 g, 296.17 mmol) were added and stirred at 70° C. After the reaction was completed, the mixture was extracted with Toluene and water, the organic layer was dried over MgSO₄ and concentrated, and the resulting compound was recrystallized using a silicagel column to obtain 43.0 g of Sub1-27 (yield: 86.7%).

### 5. Synthesis of Sub 1-33

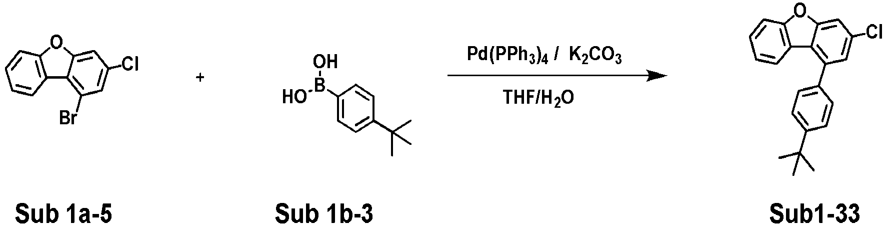

Sub 1a-5 (50 g, 177.60 mmol) and Sub 1b-3 (34.8 g, 195.36 mmol) were dissolved in THF (440) and water (150 ml) in a round-bottom flask, Pd(PPh₃)₄ (6.1 g, 5.32 mmol), K₂CO₃ (49.1 g, 355.20 mmol) were added and stirred at 70° C. After the reaction was completed, the mixture was extracted with Toluene and water, the organic layer was dried over MgSO₄ and concentrated, and the resulting compound was recrystallized using a silicagel column to obtain 46.5 of Sub1-33 (yield: 78.2%).

### 6. Synthesis of Sub 1-42

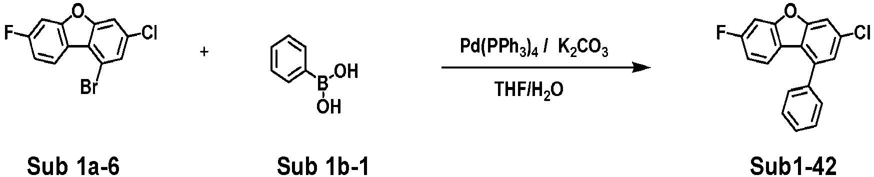

Sub 1a-6 (50 g, 166.93 mmol) and Sub 1b-1 (22.4 g, 183.63 mmol) were dissolved in THF (420) and water (140 ml) in a round-bottom flask, Pd(PPh₃)₄ (5.8 g, 5.01 mmol), K₂CO₃ (46.1 g, 333.87 mmol) were added and stirred at 70° C. After the reaction was completed, the mixture was extracted with Toluene and water, the organic layer was dried over MgSO₄ and concentrated, and the resulting compound was recrystallized using a silicagel column to obtain 42.5 of Sub1-42 (yield: 85.8%).

Meanwhile, the compounds belonging to the Sub1 may be, but are not limited to, the compounds below, and Table 1 shows the FD-MS (Field Desorption-Mass Spectrometry) values of the compounds belonging to Sub1.

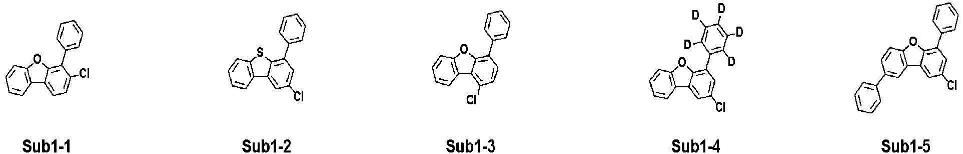

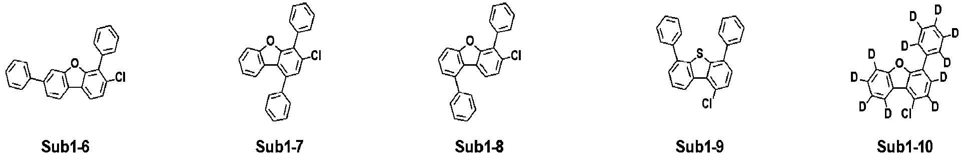

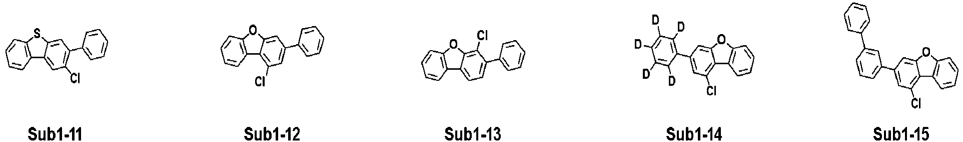

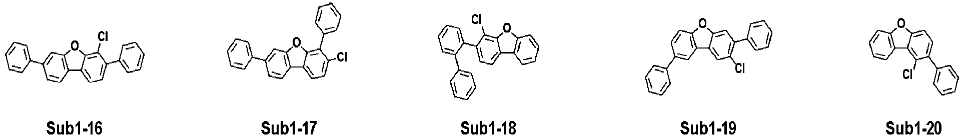

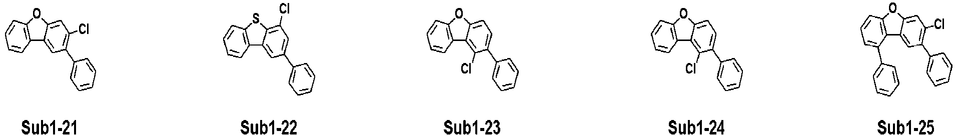

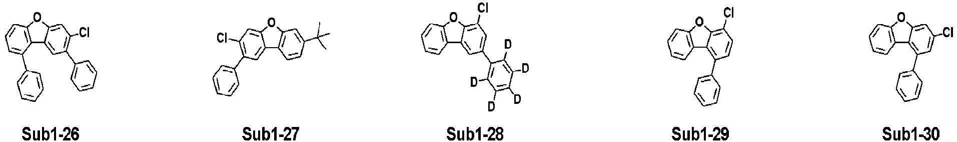

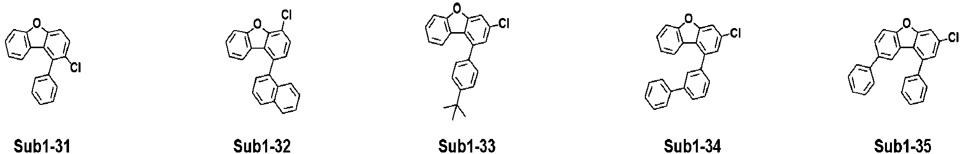

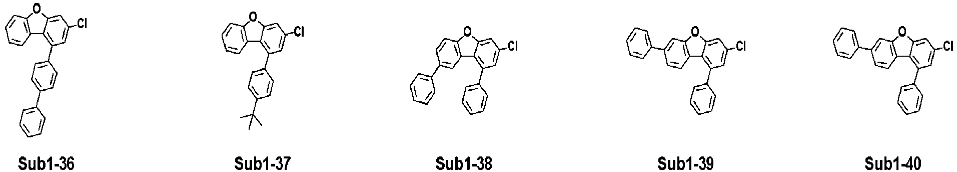

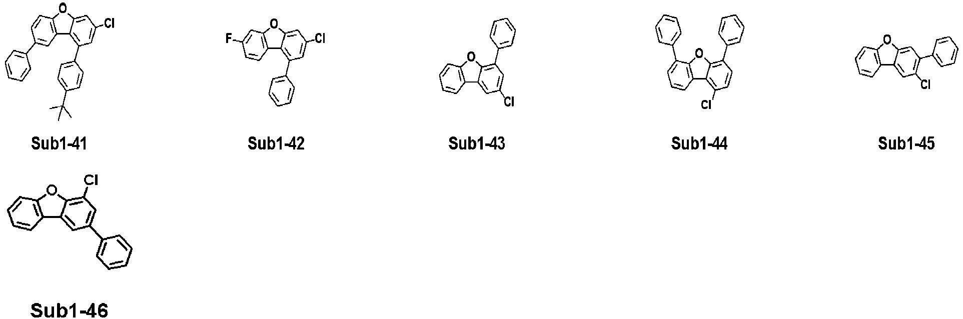

**[Table 1]**

| compound | FD-MS | compound | FD-MS |
|---|---|---|---|
| Sub1-1 | m/z=278.05(C₁₈H₁₁ClO=278.74) | Sub1-2 | m/z=294.03(C₁₈H₁₁ClS=294.80) |
| Sub1-3 | m/z=278.05(C₁₈H₁₁ClO=278.74) | Sub1-4 | m/z=283.08(C₁₈H₆D₅ClO=283.77) |
| Sub1-5 | m/z=354.08(C₂₄H₁₅ClO=354.83) | Sub1-6 | m/z=354.08(C₂₄H₁₅ClO=354.83) |
| Sub1-7 | m/z=354.08(C₂₄H₁₅ClO=354.83) | Sub1-8 | m/z=354.08(C₂₄H₁₅ClO=354.83) |
| Sub1-9 | m/z=370.06(C₂₄H₁₅ClS=370.89) | Sub1-10 | m/z=289.12(C₁₈D₁₁ClO=289.80) |
| Sub1-11 | m/z=294.03(C₁₈H₁₁ClS=294.80) | Sub1-12 | m/z=278.05(C₁₈H₁₁ClO=278.74) |
| Sub1-13 | m/z=278.05(C₁₈H₁₁ClO=278.74) | Sub1-14 | m/z=283.08(C₁₈H₆D₅ClO=283.77) |
| Sub1-15 | m/z=354.08(C₂₄H₁₅ClO=354.83) | Sub1-16 | m/z=354.08(C₂₄H₁₅ClO=354.83) |
| Sub1-17 | m/z=354.08(C₂₄H₁₅ClO=354.83) | Sub1-18 | m/z=354.08(C₂₄H₁₅ClO=354.83) |
| Sub1-19 | m/z=354.08(C₂₄H₁₅ClO=354.83) | Sub1-20 | m/z=278.05(C₁₈H₁₁ClO=278.74) |
| Sub1-21 | m/z=278. 05(C₁₈H₁₁ClO=278.74) | Sub1-22 | m/z=294.03(C₁₈H₁₁ClS=294.80) |
| Sub1-23 | m/z=278. 05(C₁₈H₁₁ClO=278.74) | Sub1-24 | m/z=278.05(C₁₈H₁₁ClO=278.74) |
| Sub1-25 | m/z=354.08(C₂₄H₁₅ClO=354.83) | Sub1-26 | m/z=354.08(C₂₄H₁₅ClO=354.83) |
| Sub1-27 | m/z=334.11(C₂₂H₁₉ClO=334.84) | Sub1-28 | m/z=283.08(C₁₈H₆D₅ClO=283.77) |
| Sub1-29 | m/z=278.05(C₁₈H₁₁ClO=278.74) | Sub1-30 | m/z=278.05(C₁₈H₁₁ClO=278.74) |
| Sub1-31 | m/z=278.05(C₁₈H₁₁ClO=278.74) | Sub1-32 | m/z=330.06(C₂₂H₁₃ClO=328.79) |
| Sub1-33 | m/z=334.11(C₂₂H₁₉ClO=334.84) | Sub1-34 | m/z=354.08(C₂₄H₁₅ClO=354.83) |
| Sub1-35 | m/z=354.08(C₂₄H₁₈ClO=354.83) | Sub1-36 | m/z=354.08(C₂₄H₁₅ClO=354.83) |
| Sub1-37 | m/z=334.11(C₂₂H₁₉ClO=334.84) | Sub1-38 | m/z=354.08(C₂₄H₁₅ClO=354.83) |
| Sub1-39 | m/z=354.08(C₂₄H₁₅ClO=354.83) | Sub1-40 | m/z=354.08(C₂₄H₁₅ClO=354.83) |
| Sub1-41 | m/z=410.14(C₂₈H₂₃ClO=410.94) | Sub1-42 | m/z=296.04(C₁₈H₁₀ClFO=296.73) |
| Sub1-43 | m/z=278.05(C₁₈H₁₁ClO=278.74) | Sub1-44 | m/z=354.08(C₂₄H₁₅ClO=354.83) |
| Sub1-45 | m/z=278.05(C₁₈H₁₁ClO=278.74) | Sub1-46 | m/z=278.05(C₁₈H₁₁ClO=278.74) |

### II. Synthesis of Sub 2

Sub2 of the reaction scheme 3 is synthesized by the reaction path of the reaction scheme 3 or reaction scheme 4, but is not limited thereto.

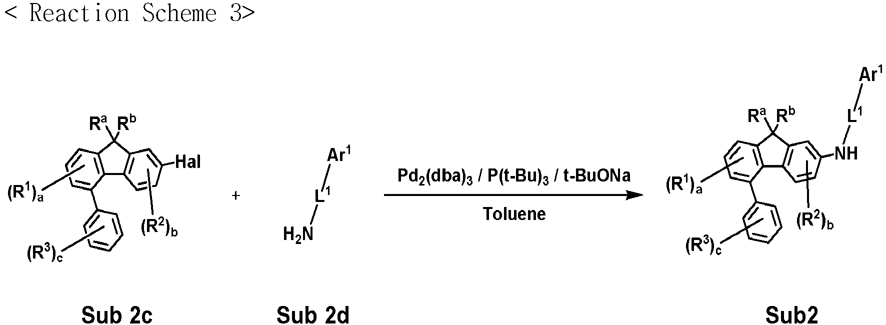

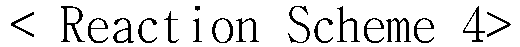

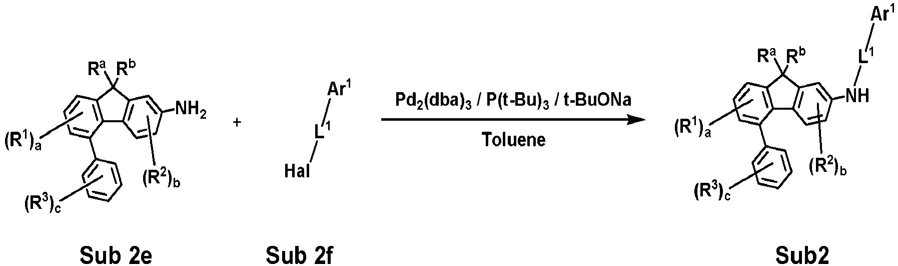

### 1. Synthesis example of Sub 2-1

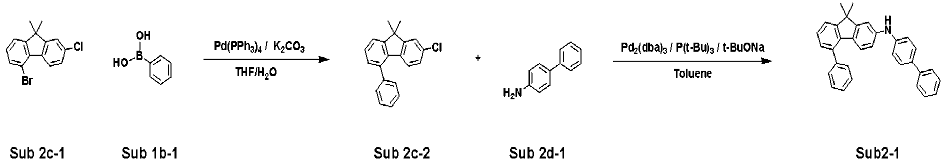

### 1) Synthesis example of Sub 2c-2

Sub 2c-1 (50 g, 162.54 mmol) and Sub 1b-1 (21.8 g, 178.80 mmol) were dissolved in THF (400) and water (130 ml) in a round-bottom flask, Pd(PPh₃)₄ (5.6 g, 4.87 mmol), K₂CO₃ (44.9 g, 325.07 mmol) were added and stirred at 60° C. After the reaction was completed, the mixture was extracted with Toluene and water, the organic layer was dried over MgSO₄ and concentrated, and the resulting compound was recrystallized using a silicagel column to obtain 42.0 of Sub2c-2 (yield: 84.7%).

### 2) Synthesis example of Sub 2-1

Sub 2c-2 (40 g, 131.22 mmol) and Sub 2d-1 (22.2 g, 131.22 mmol) were dissolved in Toluene (440 mL) in a round-bottom flask, Pd₂(dba)₃ (3.6 g, 3.94 mmol), P(t-Bu)₃ (1.59 g, 7.87 mmol), t-BuONa (25.2 g, 262.45 mmol) were added and stirred at 100° C. After the reaction was completed, the mixture was extracted with Toluene and water, the organic layer was dried over MgSO₄ and concentrated, and the resulting compound was recrystallized using a silicagel column to obtain 47.0 of Sub2-1 (yield: 81.8%).

### 2. Synthesis example of Sub 2-11

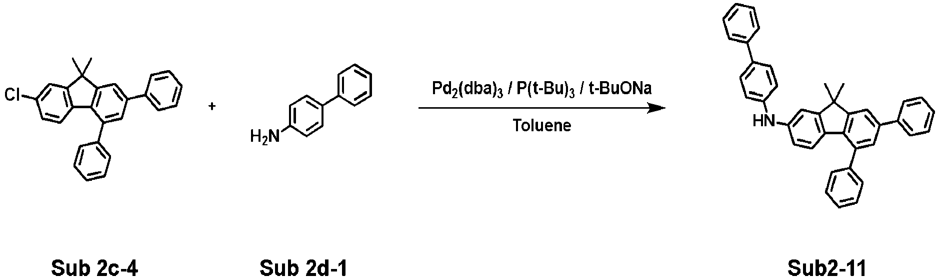

Sub 2c-4 (50 g, 131.26 mmol) and Sub 2d-1 (22.2 g, 131.26 mmol) were dissolved in Toluene (440 mL) in a round-bottom flask, Pd₂(dba)₃ (1.6 g, 3.94 mmol), P(t-Bu)₃ (1.59 g, 7.87 mmol), t-BuONa (25.2 g, 262.53 mmol) were added and 52.0 g (yield: 77.1%) of product Sub2-11 was obtained using the synthesis method of Sub2-1.

### 3. Synthesis example of Sub 2-32

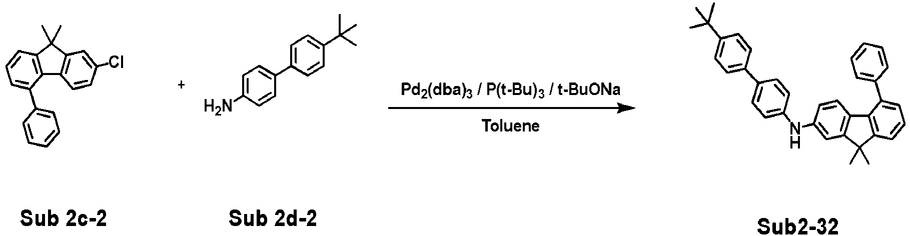

Sub 2c-2 (50 g, 164.03 mmol) and Sub 2d-2 (37.1 g, 164.03 mmol) were dissolved in Toluene (550 mL) in a round-bottom flask, Pd₂(dba)₃ (4.5 g, 4.92 mmol), P(t-Bu)₃ (2.0 g, 9.84 mmol), t-BuONa (31.5 g, 328.06 mmol) were added and 60.0 g (yield: 74.1%) of product Sub2-32 was obtained using the synthesis method of Sub2-1.

### 4. Synthesis example of Sub 2-38

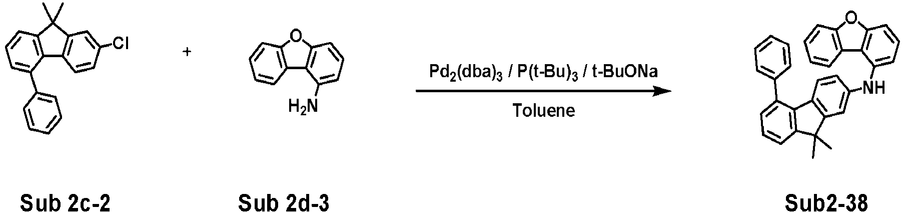

Sub 2c-2 (50 g, 164.03 mmol), Sub 2d-3 (30.0 g, 164.03 mmol) were dissolved in Toluene (550 mL) in a round-bottom flask, Pd₂(dba)₃ (4.5 g, 4.92 mmol), P(t-Bu)₃ (2.0 g, 9.84 mmol), t-BuONa (31.5 g, 328.06 mmol) were added and 66.0 g (yield: 89.1%) of product Sub2-38 was obtained using the synthesis method of Sub2-1.

### 5. Synthesis example of Sub 2-49

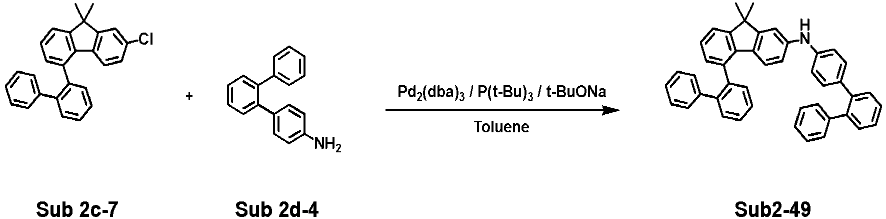

Sub 2c-7 (50 g, 131.26 mmol) and Sub 2d-4 (32.2 g, 131.26 mmol) were dissolved in Toluene (440 mL) in a round-bottom flask, Pd₂(dba)₃ (1.6 g, 3.94 mmol), P(t-Bu)₃ (1.59 g, 7.87 mmol), t-BuONa (25.2 g, 262.53 mmol) were added and 60.0 g (yield: 77.5%) of product was obtained using the synthesis method of Sub2-1.

Meanwhile, the compounds belonging to the Sub2 may be, but are not limited to, the compounds below, and Table 2 shows the FD-MS (Field Desorption-Mass Spectrometry) values of the compounds belonging to Sub2.

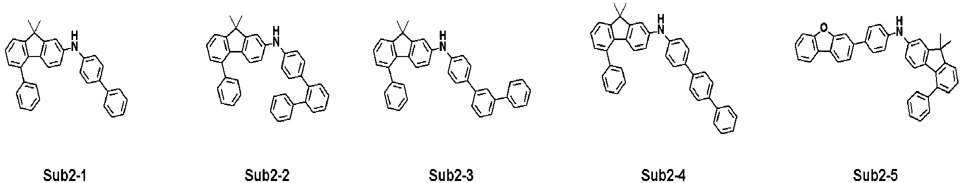

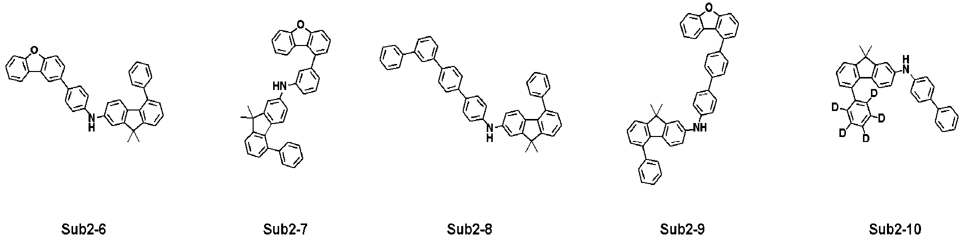

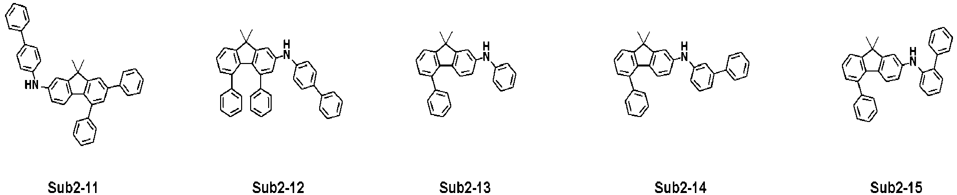

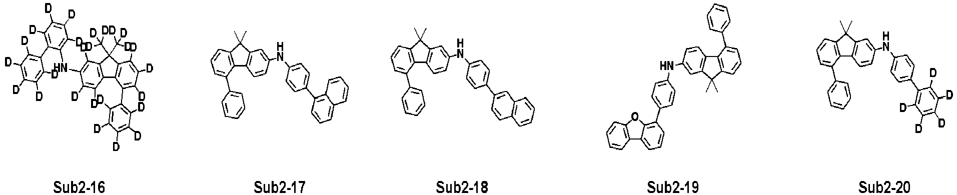

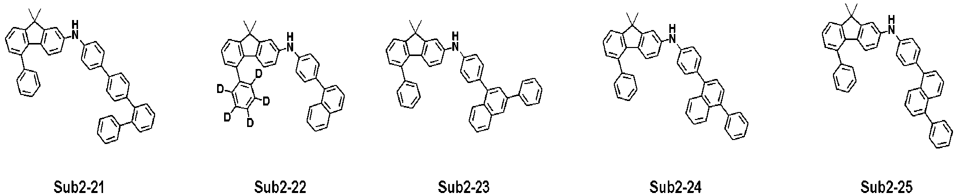

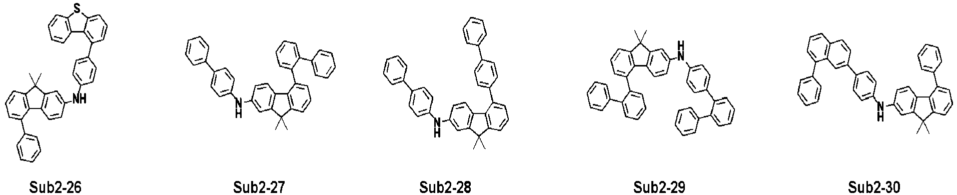

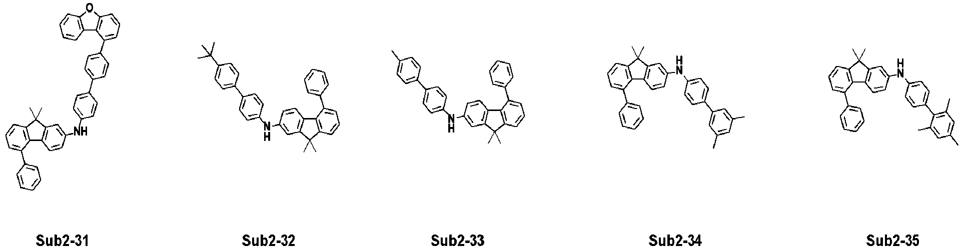

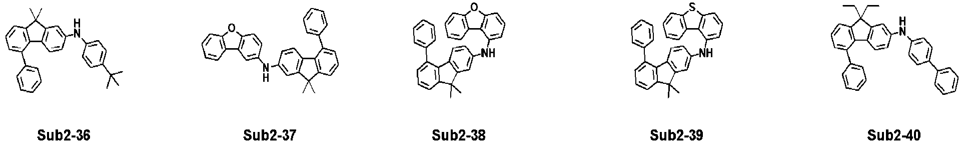

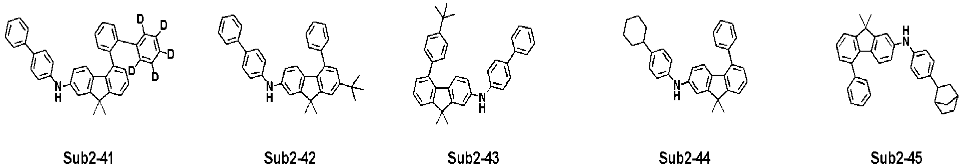

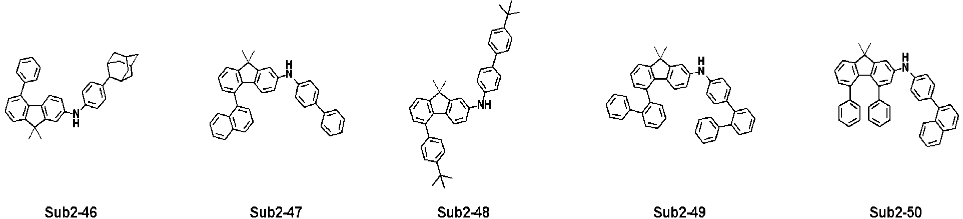

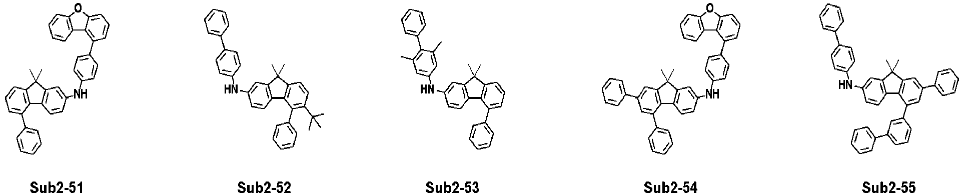

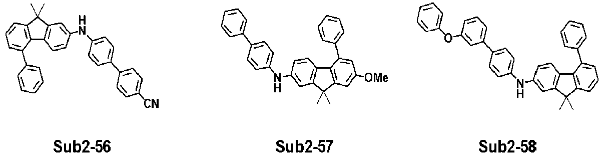

**[Table 2]**

| compound | FD-MS | compound | FD-MS |
|---|---|---|---|
| Sub2-1 | m/z=437.21(C₃₃H₂₇N=437.59) | Sub2-2 | m/z=513.25(C₃₉H₃₁N=513.68) |
| Sub2-3 | m/z=513.25(C₃₉H₃₁N=513.68) | Sub2-4 | m/z=513.25(C₃₉H₃₁N=513.68) |
| Sub2-5 | m/z=527.22(C₃₉H₂₉NO=527.67) | Sub2-6 | m/z=527.22(C₃₉H₂₉NO=527.67) |
| Sub2-7 | m/z=527.22(C₃₉H₂₉NO=527.67) | Sub2-8 | m/z=589.28(C₄₅H₃₅N=589.78) |
| Sub2-9 | m/z=603.26(C₄₅H₃₃NO=603.76) | Sub2-10 | m/z=442.25(C₃₃H₂₂D₅N=442.62) |
| Sub2-11 | m/z=513.25(C₃₉H₃₁N=513.68) | Sub2-12 | m/z=513.25(C₃₉H₃₁N=513.68) |
| Sub2-13 | m/z=361.18(C₂₇H₂₃N=361.49) | Sub2-14 | m/z=437.21(C₃₃H₂₇N=437.59) |
| Sub2-15 | m/z=437.21(C₃₃H₂₇N=437.59) | Sub2-16 | m/z=463.38(C₃₃HD₂₆N=463.74) |
| Sub2-17 | m/z=487.23(C₃₇H₂₉N=487.65) | Sub2-18 | m/z=487.23(C₃₇H₂₉N=487.65) |
| Sub2-19 | m/z=527.22(C₃₉H₂₉NO=527.67) | Sub2-20 | m/z=442.25(C₃₃H₂₂D₅N=442.62) |
| Sub2-21 | m/z=589.28(C₄₅H₃₅N=589.78) | Sub2-22 | m/z=492.26 (C₃₇H₂₄D₅N=492.68) |
| Sub2-23 | m/z=563.26(C₄₃H₃₃N=563.74) | Sub2-24 | m/z=563.26(C₄₃H₃₃N=563.74) |
| Sub2-25 | m/z=563.26(C₄₃H₃₃N=563.74) | Sub2-26 | m/z=543.20(C₃₉H₂₉NS=543.73) |
| Sub2-27 | m/z=513.25(C₃₉H₃₁N=513.68) | Sub2-28 | m/z=513.25(C₃₉H₃₁N=513.68) |
| Sub2-29 | m/z=589.28(C₄₅H₃₅N=589.78) | Sub2-30 | m/z=563.26(C₄₃H₃₃N=563.74) |
| Sub2-31 | m/z=603.26(C₄₅H₃₃NO=603,76) | Sub2-32 | m/z=493.28(C₃₇H₃₅N=493.69) |
| Sub2-33 | m/z=451.23(C₃₄H₂₉N=451.61) | Sub2-34 | m/z=465.25(C₃₅H₃₁N=465.64) |
| Sub2-35 | m/z=479.26(C₃₆H₃₃N=479.67) | Sub2-36 | m/z=417.25(C₃₁H₃₁N=417.60) |
| Sub2-37 | m/z=451.19(C₃₃H₂₅NO=451.57) | Sub2-38 | m/z=451.19(C₃₃H₂₅NO=451.57) |
| Sub2-39 | m/z=467.17(C₃₃H₂₅NS=467.63) | Sub2-40 | m/z=465.25(C₃₅H₃₁N=465.64) |
| Sub2-41 | m/z=518.28(C₃₉H₂₆D₅N=518.71) | Sub2-42 | m/z=493.28(C₃₇H₃₅N=493. 69) |
| Sub2-43 | m/z=493.28(C₃₇H₃₅N=493.69) | Sub2-44 | m/z=443.26(C₃₃H₃₃N=443.63) |
| Sub2-45 | m/z=455.26(C₃₄H₃₃N=455.64) | Sub2-46 | m/z=495.29(C₃₇H₃₇N=495.71) |
| Sub2-47 | m/z=487.23(C₃₇H₂₉N=487.65) | Sub2-48 | m/z=549.34(C₄₁H₄₃N=549.80) |
| Sub2-49 | m/z=589.28(C₄₅H₃₅N=589.78) | Sub2-50 | m/z=563.26(C₄₃H₃₃N=563.74) |
| Sub2-51 | m/z=527.22(C₃₉H₂₉NO=527.67) | Sub2-52 | m/z=493.28(C₃₇H₃₅N=493.69) |
| Sub2-53 | m/z=465.25(C₃₅H₃₁N=465.64) | Sub2-54 | m/z=603.26(C₄₅H₃₃NO=603.76) |
| Sub2-55 | m/z=589.28(C₄₅H₃₅N=589.78) | Sub2-56 | m/z=462.21(C₃₄H₂₆N₂=462.60) |
| Sub2-57 | m/z=467.22(C₃₄H₂₉NO=467.61) | Sub2-58 | m/z=529.24(C₃₉H₃₁NO=529.68) |

### III. Synthesis of Final products

### 1. Synthesis of P-1

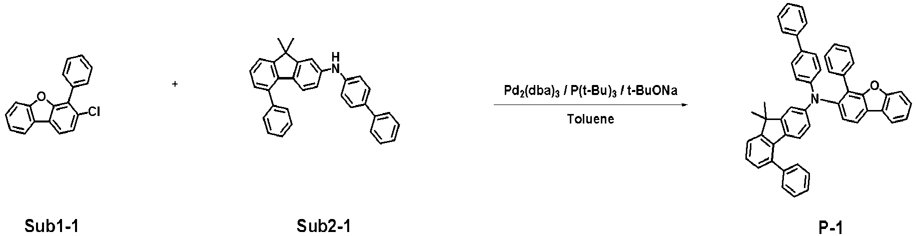

Sub1-1 (20 g, 71.75 mmol) and Sub2-1 (31.4 g, 71.7 mmol) were dissolved in Toluene (240 mL) in a round-bottom flask, Pd₂(dba)₃ (2.0 g, 2.15 mmol), P(t-Bu)₃ (0.9 g, 4.30 mmol), t-BuONa (13.8 g, 143.5 mmol) were added and stirred at 120° C. After the reaction was completed, the mixture was extracted with Toluene and water, the organic layer was dried over MgSO₄ and concentrated, and the resulting compound was recrystallized using a silicagel column to obtain 42.5 of P-1 (yield: 87.1%).

### 2. Synthesis of P-19

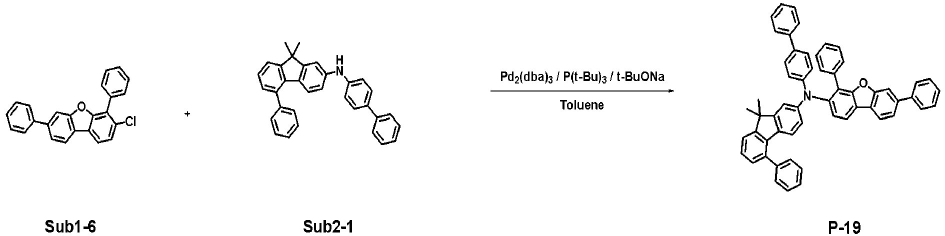

Sub1-6 (20 g, 56.36 mmol) and Sub2-1 (24.6 g, 56.36 mmol) were dissolved in Toluene (190 mL) in a round-bottom flask, Pd₂(dba)₃ (1.5 g, 1.69 mmol), P(t-Bu)₃ (0.7 g, 3.38 mmol), t-BuONa (10.8 g, 112.73 mmol) were added and 35.0 g (yield: 82.1%) of product P-19 was obtained using the synthesis method of P-1.

### 3. Synthesis of P-26

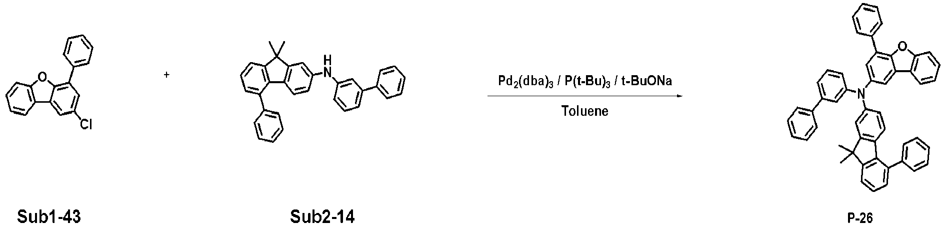

Sub1-43 (20 g, 71.75 mmol) and Sub2-14 (31.4 g, 71.75 mmol) were dissolved in Toluene (240 mL) in a round-bottom flask, Pd₂(dba)₃ (2.0 g, 2.15 mmol), P(t-Bu)₃ (0.9 g, 4.30 mmol), t-BuONa (13.8 g, 143.50 mmol) were added and 40.0 g (yield: 82.0%) of product P-26 was obtained using the synthesis method of P-1.

### 4. Synthesis of P-38

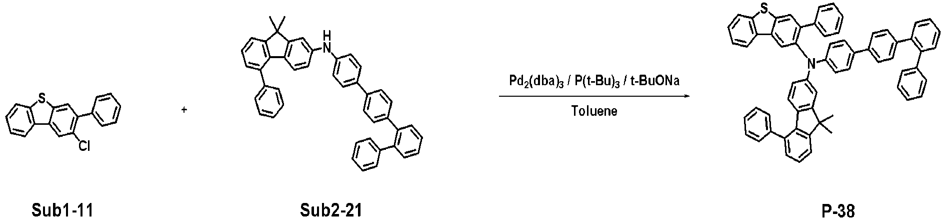

Sub1-11 (20 g, 70.22 mmol) and Sub2-21 (41.4 g, 70.22 mmol) were dissolved in Toluene (230 mL) in a round-bottom flask, Pd₂(dba)₃ (1.9 g, 2.11 mmol), P(t-Bu)₃ (0.8 g, 4.21 mmol), t-BuONa (13.5 g, 140.45 mmol) were added and 50.0 g (yield: 81.1%) of product P-38 was obtained using the synthesis method of P-1.

### 5. Synthesis of P-47

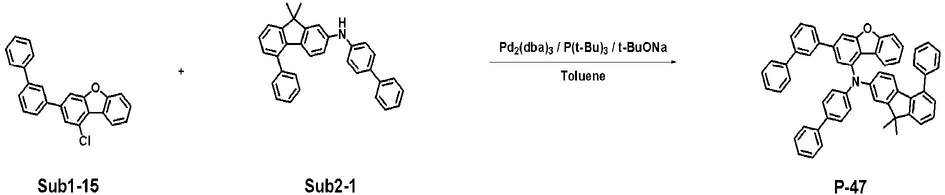

Sub1-15 (20 g, 56.36 mmol) and Sub2-1 (24.6 g, 56.36 mmol) were dissolved in Toluene (190 mL) in a round-bottom flask, Pd₂(dba)₃ (1.5 g, 1.69 mmol), P(t-Bu)₃ (0.7 g, 3.38 mmol), t-BuONa (10.8 g, 112.73 mmol) were added and 35.0 g (yield: 82.1%) of product P-47 was obtained using the synthesis method of P-1.

### 6. Synthesis of P-56

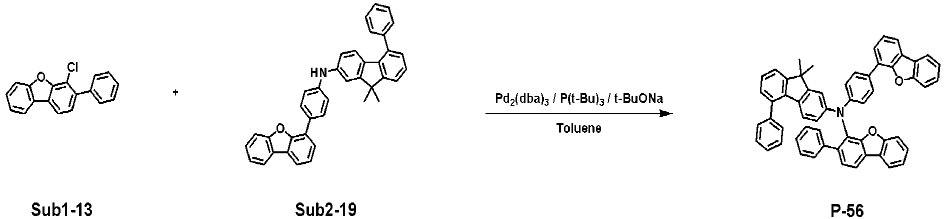

Sub1-13 (20 g, 71.75 mmol) and Sub2-19 (37.86 g, 71.75 mmol) were dissolved in Toluene (240 mL) in a round-bottom flask, Pd₂(dba)₃ (2.0 g, 2.15 mmol), P(t-Bu)₃ (0.9 g, 4.30 mmol), t-BuONa (13.8 g, 143.50 mmol) were added and 41.5 g (yield: 85.0%) of product P-56 was obtained using the synthesis method of P-1.

### 7. Synthesis of P-63

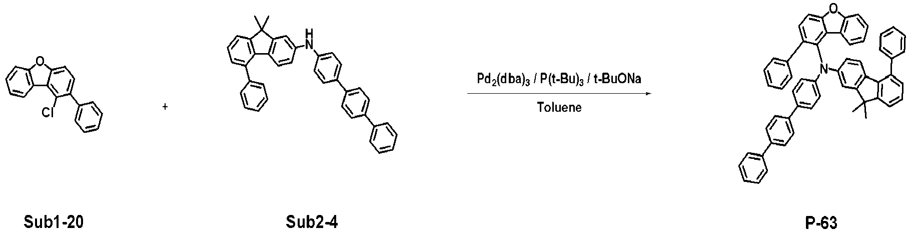

Sub1-20 (20 g, 71.75 mmol) and Sub2-4 (36.8 g, 71.75 mmol) were dissolved in Toluene (240 mL) in a round-bottom flask, Pd₂(dba)₃ (2.0 g, 2.15 mmol), P(t-Bu)₃ (0.9 g, 4.30 mmol), t-BuONa (13.8 g, 143.50 mmol) were added and 48.5 g (yield: 89.4%) of product P-63 was obtained using the synthesis method of P-1.

### 8. Synthesis of P-75

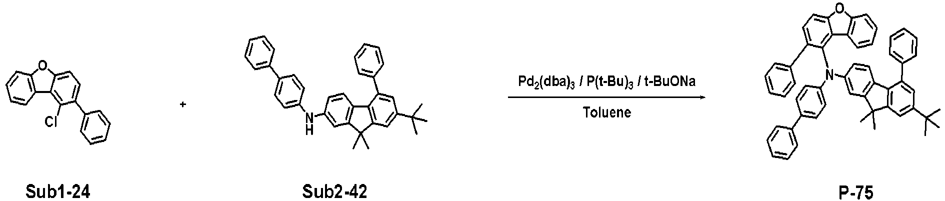

Sub1-20 (20 g, 71.75 mmol) and Sub2-4 (36.8 g, 71.75 mmol) were dissolved in Toluene (240 mL) in a round-bottom flask, Pd₂(dba)₃ (2.0 g, 2.15 mmol), P(t-Bu)₃ (0.9 g, 4.30 mmol), t-BuONa (13.8 g, 143.50 mmol) were added and 41.5 g (yield: 78.6%) of product P-75 was obtained using the synthesis method of P-1.

### 9. Synthesis of P-82

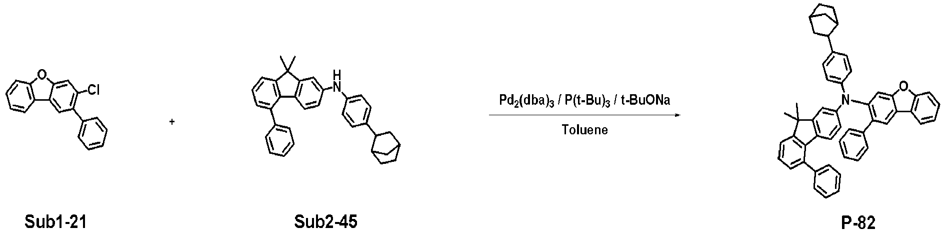

Sub1-21 (20 g, 71.75 mmol) and Sub2-45 (32.7 g, 71.7 mmol) were dissolved in Toluene (240 mL) in a round-bottom flask, Pd₂(dba)₃ (2.0 g, 2.15 mmol), P(t-Bu)₃ (0.9 g, 4.30 mmol), t-BuONa (13.8 g, 143.50 mmol) were added and 37.0 g (yield: 73.9%) of product P-82 was obtained using the synthesis method of P-1.

### 10. Synthesis of P-92

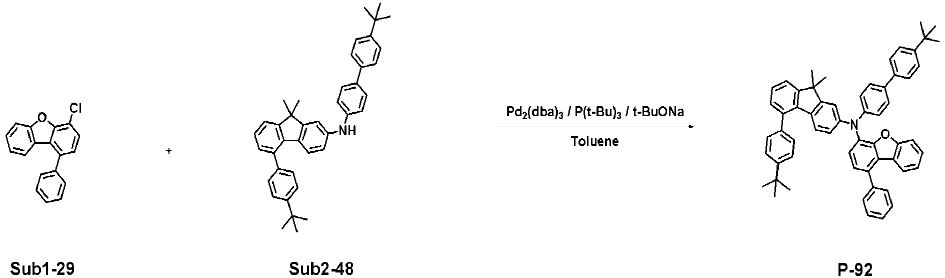

Sub1-21 (20 g, 71.75 mmol) and Sub2-45 (32.7 g, 71.7 mmol) were dissolved in Toluene (240 mL) in a round-bottom flask, Pd₂(dba)₃ (2.0 g, 2.15 mmol), P(t-Bu)₃ (0.9 g, 4.30 mmol), t-BuONa (13.8 g, 143.50 mmol) were added and 42.5 g (yield: 77.8%) of product P-92 was obtained using the synthesis method of P-1.

### 11. Synthesis of P-108

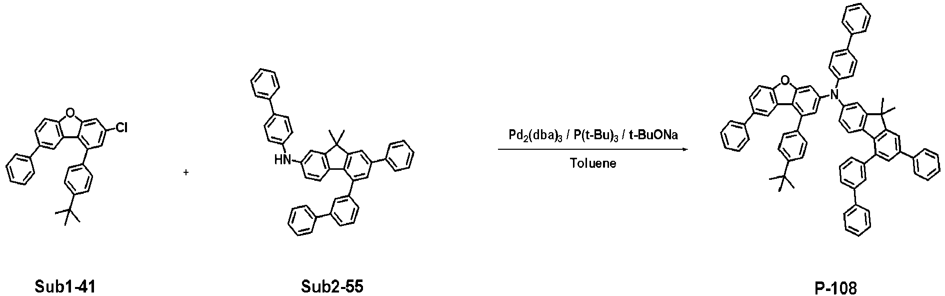

Sub1-21 (20 g, 71.75 mmol), Sub2-45 (32.7 g, 71.7 mmol) were dissolved in Toluene (240 mL) in a round-bottom flask, Pd₂(dba)₃ (2.0 g, 2.15 mmol), P(t-Bu)₃ (0.9 g, 4.30 mmol), t-BuONa (13.8 g, 143.50 mmol) were added and 34.5 g (yield: 73.5%) of product P-108 was obtained using the synthesis method of P-1.

Meanwhile, the FD-MS values of compounds P-1 to P-120 of the present invention manufactured according to the above-described synthetic examples are as shown in Table 3.

**[Table 3]**

| compound | FD-MS | compound | FD-MS |
|---|---|---|---|
| P-1 | m/z=679.29(C₅₁H₃₇NO=679.86) | P-2 | m/z=695.26(C₅₁H₃₇NS=695.92) |
| P-3 | m/z=679.29(C₅₁H₃₇NO=679.86) | P-4 | m/z=755.32(C₅₇H₄₁NO=755.96) |
| P-5 | m/z=755.32(C₅₇H₄₁NO=755.96) | P-6 | m/z=755.32(C₅₇H₄₁NO=755.96) |
| P-7 | m/z=769.30(C₅₇H₃₉NO₂=769.94) | P-8 | m/z=769.30(C₅₇H₃₉NO₂=769.94) |
| P-9 | m/z=755.32(C₅₇H₄₁NO=755.96) | P-10 | m/z=769.30(C₅₇H₃₉NO₂=769.94) |
| P-11 | m/z=769.30(C₅₇H₃₉NO₂=769.94) | P-12 | m/z=831.35(C₆₃H₄₅NO=832.06) |
| P-13 | m/z=845.33(C₆₃H₄₃NO₂=846.04) | P-14 | m/z=684.32(C₅₁H₃₂D₅NO=684.89) |
| P-15 | m/z=684.32(C₅₁H₃₂D₅NO=684.89) | P-16 | m/z=689.35(C₅₁H₂₇D₁₀NO=689.92) |
| P-17 | m/z=755.32(C₅₇H₄₁NO=755.96) | P-18 | m/z=755.32(C₅₇H₄₁NO=755. 96) |
| P-19 | m/z=755.32(C₅₇H₄₁NO=755.96) | P-20 | m/z=755.32(C₅₇H₄₁NO=755. 96) |
| P-21 | m/z=755.32(C₅₇H₄₁NO=755.96) | P-22 | m/z=755.32(C₅₇H₄₁NO=755. 96) |
| P-23 | m/z=771.30(C₅₇H₄₁NS=772.02) | P-24 | m/z=755.32(C₅₇H₄₁NO=755.96) |
| P-25 | m/z=603.26(C₄₅H₃₃NO=603.76) | P-26 | m/z=679.29(C₅₁H₃₇NO=679.86) |
| P-27 | m/z=679.29(C₅₁H₃₇NO=679.86) | P-28 | m/z=716.52(C₅₁D₃₇NO=717.09) |
| P-29 | m/z=679.29(C₅₁H₃₇NO=679.86) | P-30 | m/z=679.29(C₅₁H₃₇NO=679.86) |
| P-31 | m/z=679.29(C₅₁H₃₇NO=679.86) | P-32 | m/z=755.32(C₅₇H₄₁NO=755.96) |
| P-33 | m/z=755.32(C₅₇H₄₁NO=755.96) | P-34 | m/z=734.33(C₅₅H₃₄D₅NO=734.95) |
| P-35 | m/z=729.30(C₅₅H₃₉NO=729.92) | P-36 | m/z=769.30(C₅₇H₃₉NO₂=769.94) |
| P-37 | m/z=684.32(C₅₁H₃₂D₅NO=684.89) | P-38 | m/z=847.33(C₆₃H₄₅NS=848.12) |
| P-39 | m/z=729.30(C₅₅H₃₉NO=729.92) | P-40 | m/z=734.33(C₅₅H₃₄D₅NO=734.96) |
| P-41 | m/z=805.33(C₆₁H₄₃NO=806.02) | P-42 | m/z=805.33(C₆₁H₄₃NO=806.02) |
| P-43 | m/z=805.33(C₆₁H₄₃NO=806.02) | P-44 | m/z=785.28(C₅₇H₃₉NOS=786.00) |
| P-45 | m/z=755.32(C₅₇H₄₁NO=755.96) | P-46 | m/z=755.32(C₅₇H₄₁NO=755.96) |
| P-47 | m/z=755.32(C₅₇H₄₁NO=755.96) | P-48 | m/z=755.32(C₅₇H₄₁NO=755.96) |
| P-49 | m/z=755.32(C₅₇H₄₁NO=755.96) | P-50 | m/z=831.35(C₆₃H₄₅NO=832.06) |
| P-51 | m/z=831.35(C₆₃H₄₅NO=832.06) | P-52 | m/z=907.38(C₆₉H₄₉NO=908.16) |
| P-53 | m/z=729.30(C₅₅H₃₉NO=729.92) | P-54 | m/z=755.32(C₅₇H₄₁NO=755.96) |
| P-55 | m/z=811.38(C₆₁H₄₉NO=812.07) | P-56 | m/z=769.30(C₅₇H₃₉NO₂=769.94) |
| P-57 | m/z=679.29(C₅₁H₃₇NO=679.86) | P-58 | m/z=679.29(C₅₁H₃₇NO=679. 86) |
| P-59 | m/z=679.29(C₅₁H₃₇NO=679.86) | P-60 | m/z=729.30(C₅₅H₃₉NO=729.92) |
| P-61 | m/z=805.33(C₆₁H₄₃NO=806.02) | P-62 | m/z=769.30(C₅₇H₃₉NO₂=769.94) |
| P-63 | m/z=755.32(C₅₇H₄₁NO=755.96) | P-64 | m/z=845.33(C₆₃H₄₃NO₂=846.04) |
| P-65 | m/z=735.35(C₅₅H₄₅NO=735.97) | P-66 | m/z=693.30(C₅₂H₃₉NO=6393.89) |
| P-67 | m/z=707.32(C₅₃H₄₁NO=707.92) | P-68 | m/z=721.33(C₅₄H₄₃NO=721.94) |
| P-69 | m/z=659.32(C₄₉H₄₁NO=659.87) | P-70 | m/z=709.24(C₅₁H₃₅NOS=709.91) |
| P-71 | m/z=693.27(C₅₁H₃₅NO₂=693.85) | P-72 | m/z=709.24(C₅₁H₃₅NOS=709.91) |
| P-73 | m/z=707.32(C₅₃H₄NO=707.92) | P-74 | m/z=760.35(C₅₇H₃₆D₅NO=760.99) |
| P-75 | m/z=735.35(C₅₅H₄₅NO=735.97) | P-76 | m/z=755.32(C₅₇H₄₁NO=755.96) |
| P-77 | m/z=845.33(C₆₃H₄₃NO₂=846.04) | P-78 | m/z=811.38(C₆₁H₄₉NO=812.07) |
| P-79 | m/z=735.35(C₅₅H₄₅NO=735.97) | P-80 | m/z=740.38(C₅₅H₄₀D₅NO=741.00) |
| P-81 | m/z=685.33(C₅₁H₄₃NO=685.91) | P-82 | m/z=697.33(C₅₂H₄₃NO=697.92) |
| P-83 | m/z=737.37(C₅₅H₄₇NO=737.99) | P-84 | m/z=685.33(C₅₁H₄₃NO=685.91) |
| P-85 | m/z=679.29(C₅₁H₃₇NO=679.86) | P-86 | m/z=679.29(C₅₁H₃₇NO=679.86) |
| P-87 | m/z=679.29(C₅₁H₃₇NO=679.86) | P-88 | m/z=769.30(C₅₇H₃₉NO₂=769.94) |
| P-89 | m/z=755.32(C₅₇H₄₁NO=755.96) | P-90 | m/z=729.30(C₅₅H₃₉NO=729.92) |
| P-91 | m/z=729.30(C₅₅H₃₉NO=729.92) | P-92 | m/z=791.41(C₅₉H₅₃NO=792.08) |
| P-93 | m/z=791.41(C₅₉H₅₃NO=792.08) | P-94 | m/z=785.37(C₅₉H₄₇NO=786.03) |
| P-95 | m/z=755.32(C₅₇H₄₁NO=755.96) | P-96 | m/z=831.35(C₆₃H₄₅NO=832.06) |
| P-97 | m/z=783.35(C₅₉H₄₅NO=784.01) | P-98 | m/z=831.35(C₆₃H₄₅NO=832.06) |
| P-99 | m/z=805.33(C₆₁H₄₃NO=806.02) | P-100 | m/z=845.33(C₆₃H₄₃NO₂=846.04) |
| P-101 | m/z=679.29(C₅₁H₃₇NO=679.86) | P-102 | m/z=735.35(C₅₅H₄₅NO=735.97) |
| P-103 | m/z=769.30(C₅₇H₃₉NO₂=769.94) | P-104 | m/z=763.38(C₅₇H₄₉NO=764.02) |
| P-105 | m/z=755.32(C₅₇H₄₁NO=755.96) | P-106 | m/z=811.38(C₆₁H₄₉NO=812.07) |
| P-107 | m/z=921.36(C₆₉H₄₇NO₂=922.14) | P-108 | m/z=963.44(C₇₃H₅₇NO=964.26 |
| P-109 | m/z=704.28(C₅₂H₃₆N₂O=704.87) | P-110 | m/z=697.28(C₅₁H₃₆FNO=687.85) |
| P-111 | m/z=709.49(C₅₂H₃₉NO₂=709.30) | P-112 | m/z=771.31(C₅₇H₄₁NO₂=771.96) |
| P-113 | m/z=893.33(C₆₇H₄₃NO₂=894.09) | P-114 | m/z=790.31(C₅₇H₃₄D₅NOS=791.04) |
| P-115 | m/z=769.30(C₅₇H₃₉NO₂=769.94) | P-116 | m/z=679.29(C₅₁H₃₇NO=679.86) |
| P-117 | m/z=719.32(C₅₄H₄₁NO=719.93) | P-118 | m/z=719.32(C₅₄H₄₁NO=719.93) |
| P-119 | m/z=719.32(C₅₄H₄₁NO=719.93) | P-120 | m/z=699.30(C₅₁H₂₉D₆NO₂=699.88) |

Meanwhile, exemplary synthesis examples of the present invention represented by Formula (1) have been described, but these are all based on the Buchwald-Hartwig cross coupling reaction, Miyaura boration reaction, Suzuki cross-coupling reaction, Intramolecular acid-induced cyclization reaction (J. mater. Chem.1999, 9, 2095.), Pd(II)-catalyzed oxidative cyclization reaction *(*Org. Lett.2011, 13, 5504), and PPh₃-mediated reductive cyclization reaction (J. Org. Chem. 2005, 70, 5014.), and it will be easily understood by those skilled in the art that the reaction proceeds even when other substituents defined in Formula (1) are bonded in addition to the substituents specified in the specific synthesis examples.

### Manufacturing Evaluation of Organic Electroluminescent Devices

### [Example 1] Green organic light-emitting device (emitting-auxiliary Layer)

An organic light-emitting device was manufactured using the compound of the present invention as an emitting auxiliary layer material according to a conventional method. First, N¹-(naphthalen-2-yl)-N⁴,N⁴-bis(4-(naphthalen-2-yl(phenyl)amino)phenyl)-N¹-phenylbenzene-1,4-diamine (hereinafter abbreviated as 2-TNATA) film a was vacuum-deposited as a hole injection layer on the ITO layer (anode) formed on a glass substrate to a thickness of 60 nm. Next, N,N'-Bis(1-naphthalenyl)-N,N'-bis-phenyl-(1,1'-biphenyl)-4,4'-diamine (hereinafter abbreviated as NPB) was vacuum-deposited to a thickness of 60 nm to form a hole transport layer. Next, a compound represented by C-1 was vacuum-deposited to a thickness of 30 nm on the hole transport layer to form a buffer layer, and a compound P-1 of the present invention represented by Formula was vacuum-deposited to a thickness of 7 nm on the buffer layer to form an emitting auxiliary layer. After forming the emitting auxiliary layer, 4,4'-N,N'-dicabazole-biphenyl (hereinafter abbreviated as CBP) was used as a host material and [tris(2-phenylpyridine)-iridium] (hereinafter abbreviated as Ir(ppy)₃) was used as a dopant material on the emitting auxiliary layer, and the doping was done in a weight ratio of 95:5, and vacuum-deposited to a thickness of 30 nm to form an emitting layer. Next, (1,1'-bisphenyl)-4-oleato)bis(2-methyl-8-quinolinolato)aluminum
(hereinafter abbreviated as BAlq) was vacuum-deposited to a thickness of 5 nm as a hole-blocking layer, and tris(8-quinolinol)aluminum (hereinafter abbreviated as Alq₃) was deposited to a thickness of 40 nm as an electron transport layer. Afterwards, LiF, a halogenated alkali metal, was deposited as an electron injection layer with a thickness of 0.2 nm, and Al was then deposited with a thickness of 150 nm and used as a cathode, thereby manufacturing an organic light-emitting device.

### [Example 2] to [Example 16] Green organic light-emitting device (emitting-auxiliary Layer)

An organic light-emitting device was manufactured in the same manner as in Example 1, except that the compounds P-2 to P-119 of the present invention described in Table 4 were used instead of the compound P-1 of the present invention as an emitting auxiliary layer material.

### [Comparative Example 1] to [Comparative Example 3]

An organic light-emitting device was manufactured in the same manner as Example 1, except that Comparative Compounds A to C were used as an emitting-auxiliary layer material.

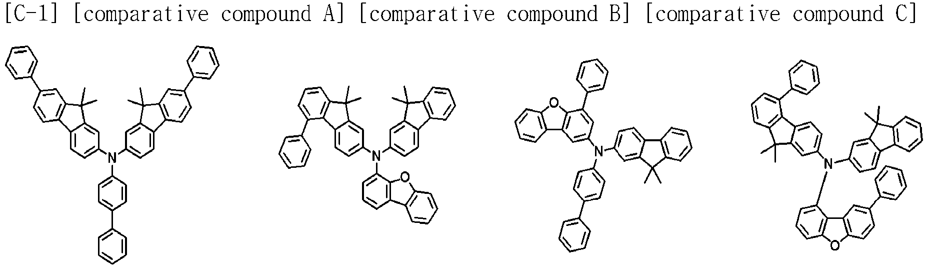

The electroluminescence (EL) characteristics were measured using PR-650 from Photoresearch by applying a forward bias DC voltage to the organic light-emitting devices and the comparative examples manufactured in this way. As a result of the measurement, the T95 lifespan was measured using a lifespan measuring device manufactured by Max Science at a standard brightness of 5,000 cd/m². Table 4 shows the results of the device fabrication and evaluation.

The measuring apparatus can evaluate the performance of new materials compared to comparative compounds under identical conditions, without being affected by possible daily fluctuations in deposition rate, vacuum quality or other parameters.

Since, during the evaluation, one batch contains 4 identically prepared OLEDs including a comparative compound, and the performance of a total of 12 OLEDs in 3 batches is **evaluated individually,** the value of the experimental results obtained in this way indicates statistical significance.

**[Table 4]**

| | compound | Driving Voltage (V) | Current Density (mA/cm²) | Efficiency (cd/A) | T(95) |
|---|---|---|---|---|---|
| comparative example(1) | comparative compound A | 5.1 | 16.0 | 31.2 | 110.2 |
| comparative example(2) | comparative compound B | 5.2 | 13.9 | 35.9 | 128.6 |
| comparative example(3) | comparative compound C | 5.1 | 13.7 | 36.4 | 130.1 |
| example(1) | compound(P-1) | 5.0 | 12.5 | 40.0 | 137.4 |
| example(2) | compound(P-2) | 4.7 | 12.5 | 40.2 | 137.5 |
| example(3) | compound(P-3) | 4.9 | 11.8 | 42.5 | 148.9 |
| example(4) | compound(P-15) | 4.9 | 12.2 | 41.1 | 150.9 |
| example(5) | compound(P-26) | 4.8 | 12.2 | 40.9 | 141.9 |
| example(6) | compound(P-29) | 5.0 | 12.3 | 40.8 | 139.9 |
| example(7) | compound(P-31) | 5.0 | 12.5 | 40.2 | 138.1 |
| example(8) | compound (P-65) | 5.0 | 12.4 | 40.5 | 139.0 |
| example(9) | compound (P-83) | 5.0 | 12.3 | 40.7 | 139.6 |
| example(10) | compound (P-86) | 4.7 | 12.0 | 41.5 | 144.7 |
| example(11) | compound(P-97) | 4.7 | 12.1 | 41.4 | 144.5 |
| example(12) | compound(P-115) | 4.9 | 11.9 | 42.0 | 150.7 |
| example(13) | compound(P-116) | 5.0 | 12.2 | 40.8 | 140.1 |
| example(14) | compound(P-117) | 4.8 | 12.2 | 41.1 | 145.4 |
| example(15) | compound(P-118) | 4.7 | 12.6 | 39.6 | 138.6 |
| example(16) | compound(P-119) | 4.6 | 12.5 | 40.2 | 141.3 |

As can be seen from the results in Table 4, when a green organic light-emitting device is manufactured using the material for an organic light-emitting device of the present invention as an emitting auxiliary layer material, the light-emitting efficiency and lifespan of the organic light-emitting device can be significantly improved compared to the comparative examples using comparative compounds A to C.

Comparing the comparative compound A with the compound of the present invention, the compound of the present invention differs in that an aryl group containing a phenyl group is further substituted on dibenzofuran or dibenzothiophene. Additionally, when comparing the comparative compound B with the compound of the present invention, the compound of the present invention differs in that an aryl group including a phenyl group is further substituted at the 5-position of fluorene. Comparing the comparative compound C and the compound of the present invention, there is a difference in that the aryl group is substituted in dibenzofuran or dibenzothiophene, but the position is different.

As a result, it can be confirmed that the device results of Examples 1 to 16 manufactured using the compound of the present invention represented by Formula (1) show significantly superior results, and it can be confirmed that the device performance is superior to that of other comparative compounds not described in this specification.

Table 5 shows data measured using the DFT Method (B3LYP/6-31(D)) of the Gaussian program for comparative compounds A to C and compound P-29 of the present invention.

**[Table 5]**

| | comparative compound A | comparative compound B | comparative compound C | P-29 |
|---|---|---|---|---|
| G.HOMO | 4.742 | 4.793 | 4.798 | 4.894 |

As can be seen in Table 5, it can be seen that compound P-29 of the present invention has a deeper HOMO value than comparative compounds A to C. This brings it close to the HOMO of the host, which allows for smooth hole injection from the emitting auxiliary layer to the host, and therefore, the efficiency and lifespan of the device appear to increase significantly due to smooth hole injection. That is, as can be seen above, there may be differences in the physical properties of the compound depending on the structure of the compound, and even if the structure is similar, it appears that the presence or absence, type, and location of the substituent have a great influence on the characteristics of the device.

Additionally, the evaluation results of the above-described device fabrication described the device characteristics in which the compound of the present invention was applied only to the emitting auxiliary layer, but the compound of the present invention may be applied to the hole transport layer or the buffer layer, or may be applied to all of the hole transport layer, the buffer layer, and the emitting auxiliary layer.

Although exemplary embodiments of the present invention have been described for illustrative purposes, those skilled in the art will appreciate that various modifications, additions and substitutions are possible, without departing from the scope and spirit of the invention as disclosed in the accompanying claims. Therefore, the embodiment disclosed in the present invention is intended to illustrate the scope of the technical idea of the present invention, and the scope of the present invention is not limited by the embodiment. The scope of the present invention shall be construed on the basis of the accompanying claims, and it shall be construed that all of the technical ideas included within the scope equivalent to the claims belong to the present invention.

### [Industrial Applicability]

According to the present invention, organic devices with excellent device characteristics such as high brightness, high luminescence, and long lifespan can be manufactured, thereby demonstrating industrial applicability.

## Claims

1. A compound represented by Formula (1): 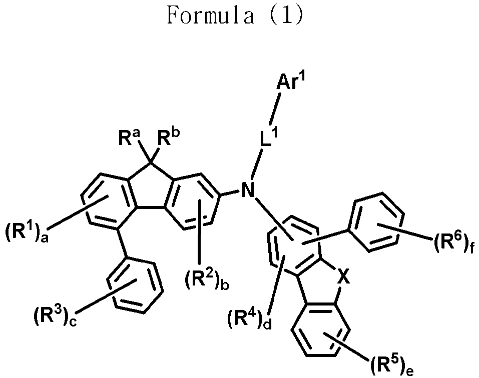 wherein:
1) X is 0 or S,
2) Ar¹ is selected from the group consisting of a C₆-C₆₀ aryl group; a fluorenyl group; a C₂-C₆₀ heterocyclic group including at least one heteroatom of 0, N, S, Si and P; and a C₃-C₆₀ aliphatic ring;
3) L¹ is independently a single bond; a C₆-C₆₀ arylene group; or a C₂-C₆₀ heterocyclic group;
4) R¹, R², R³, R⁴, R⁵ and R⁶ are independently the same or different from each other and are independently selected from the group consisting of hydrogen; deuterium; halogen; a C₁-C₅₀ alkyl group; a C₂-C₂₀ alkenyl group; a C₂-C₂₀ alkynyl group; a C₁-C₃₀ alkoxyl group; and a C₆-C₃₀ an aryloxy group; a C₆-C₆₀ aryl group; and a C₂-C₆₀ heterocyclic group;
5) R^{a} and R^{b} are independently a C₁-C₅₀ alkyl group; or a C₆-C₆₀ aryl group,
6) a and b are independently an integer of 0 to 3, d is an integer of 0 to 2, e is an integer of 0 to 4, c and f are independently an integer of 0 to 5,
wherein the aryl group, arylene group, heterocyclic group, fluorenyl group, aliphatic ring group, alkyl group, alkenyl group, alkynyl group, alkoxyl group and aryloxy group may be substituted with one or more substituents selected from the group consisting of deuterium; halogen; silane group; siloxane group; boron group; germanium group; a cyano group; a nitro group; a C₁-C₂₀ alkylthio group; a C₁-C₂₀ alkoxyl group; a C₆-C₂₀ aryloxy group; a C₁-C₂₀ alkyl group; a C₂-C₂₀ alkenyl group; a C₂-C₂₀ alkynyl group; a C₆-C₂₀ aryl group; a C₆-C₂₀ aryl group substituted with deuterium; a fluorenyl group; a C₂-C₂₀ heterocyclic group; a C₃-C₂₀ aliphatic ring; a C₇-C₂₀ arylalkyl group; a C₆-C₂₀ arylalkenyl group; and a C₇-C₂₀ alkylaryl group; and the hydrogen of these substituents may be further substituted with one or more deuterium, and the substituents may be bonded to each other to form a saturated or unsaturated ring, wherein the term 'ring' means a C₃-C₆₀ aliphatic ring or a C₆-C₆₀ aromatic ring or a C₂-C₅₀ heterocyclic group or a fused ring formed by the combination thereof.

2. The compound according to claim 1, wherein Formula (1) is represented by any one of Formulas (2) to (5): 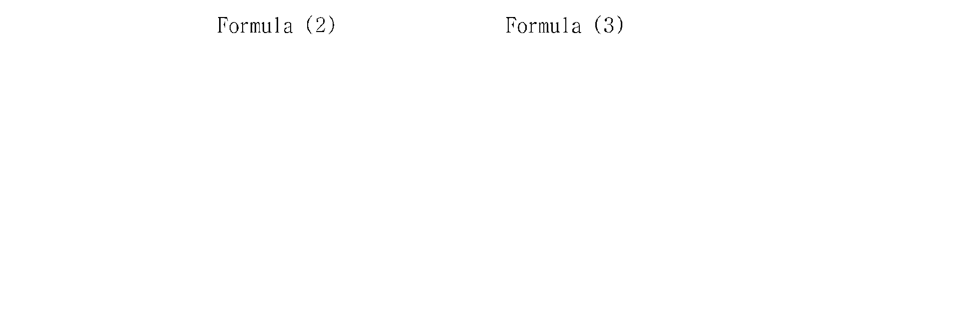 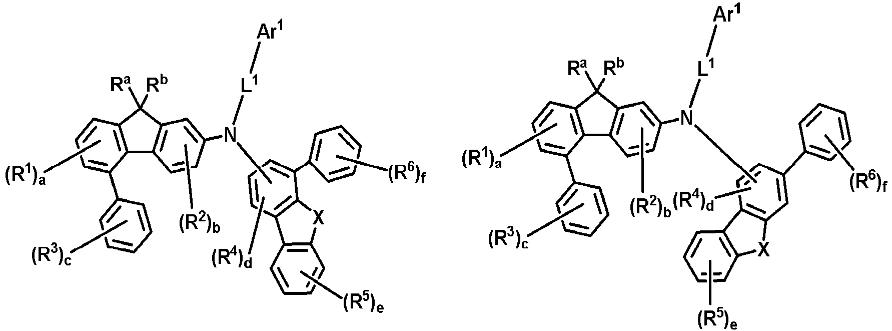 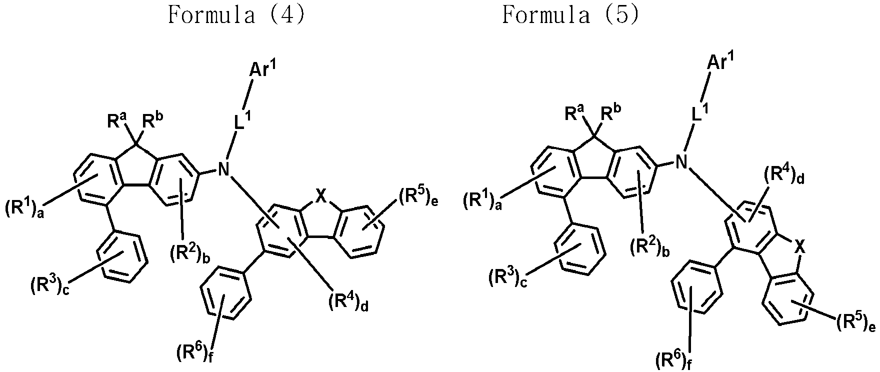 wherein, X, Ar¹, L¹, R¹, R², R³, R⁴, R⁵, R⁶, R^{a}, R^{b}, a, b, c, d, e and f are the same as defined in claim 1.

3. The compound according to claim 1, wherein Formula (1) is represented by any one of Formulas (6) to (9): 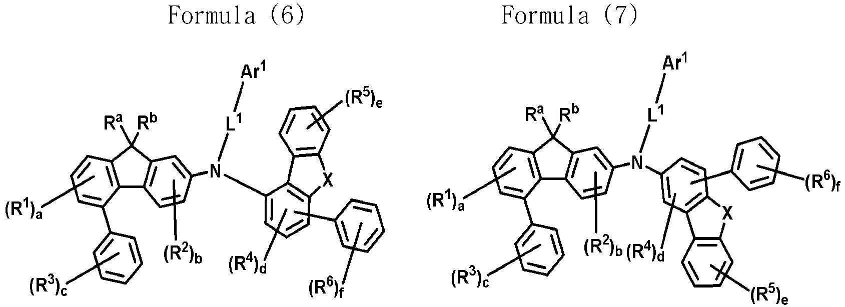 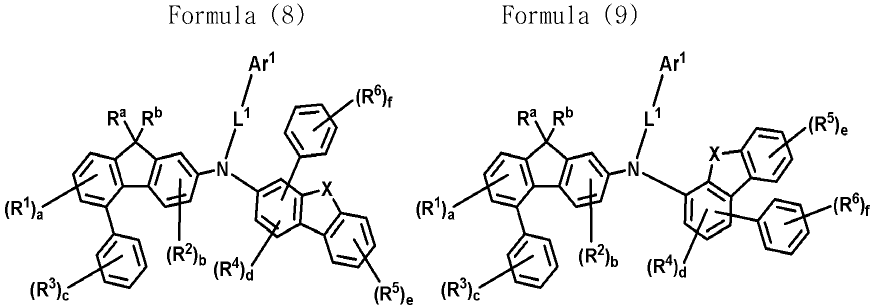 wherein, X, Ar¹, L¹, R¹, R², R³, R⁴, R⁵, R⁶, R^{a}, R^{b}, a, b, c, d, e and f are the same as defined in claim 1.

4. The compound according to claim 1, wherein Formula (1) is represented by any one of Formulas (10) to (15): 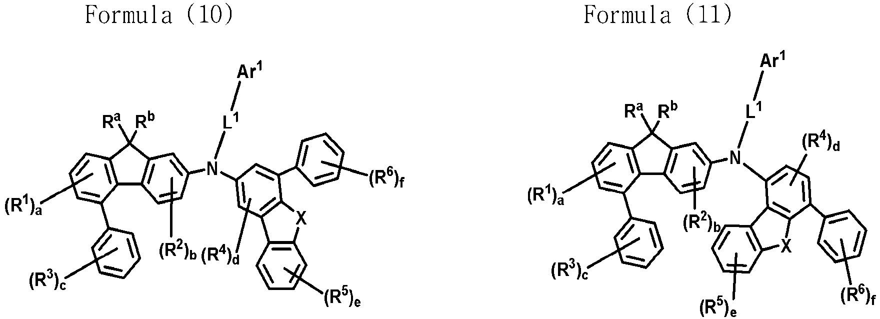 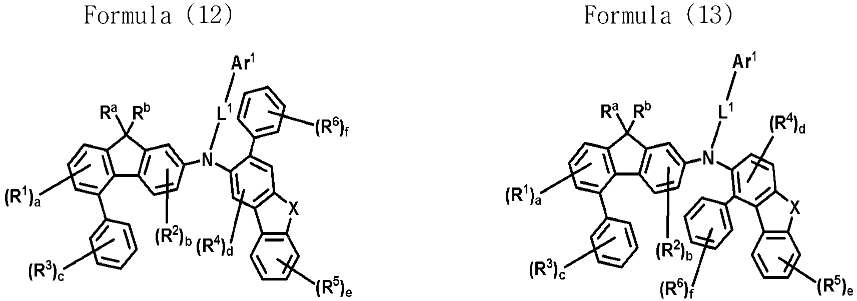 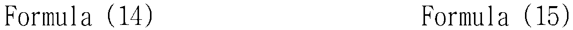 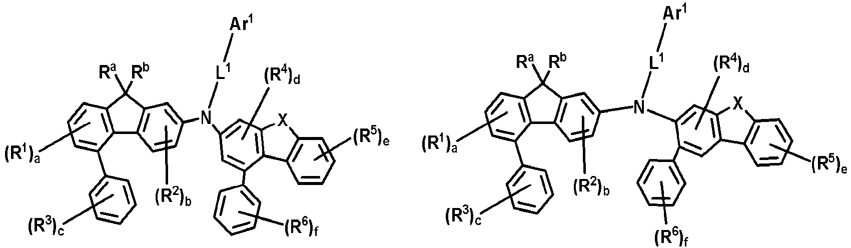 wherein, X, Ar¹, L¹, R¹, R², R³, R⁴, R⁵, R⁶, R^{a}, R^{b}, a, b, c, d, e and f are the same as defined in claim 1.

5. The compound according to claim 1, wherein the compound represented by Formula (1) may be represented by any one of the following compounds P-1 to P-120.

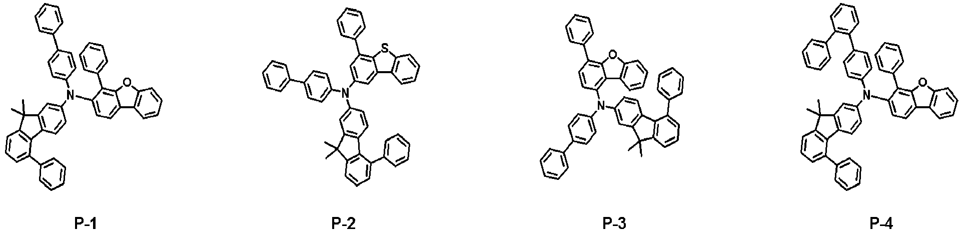

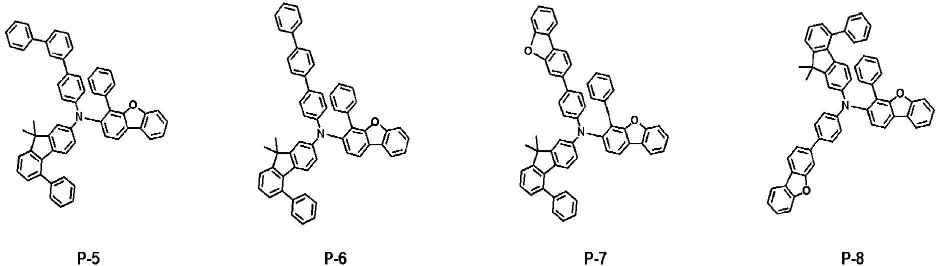

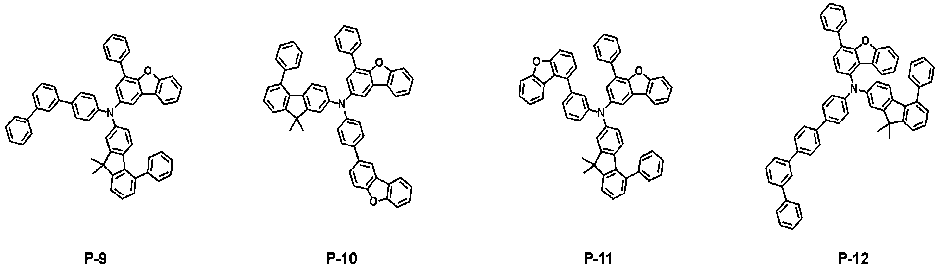

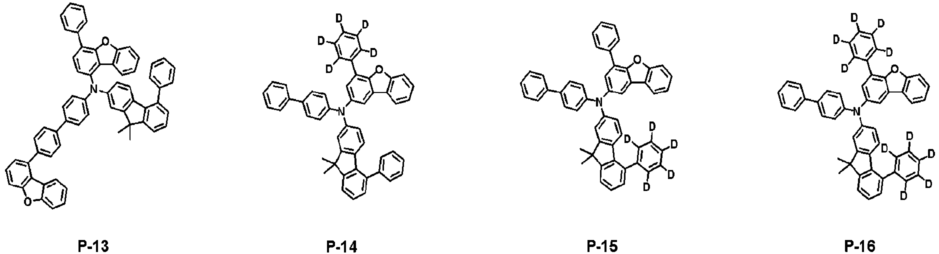

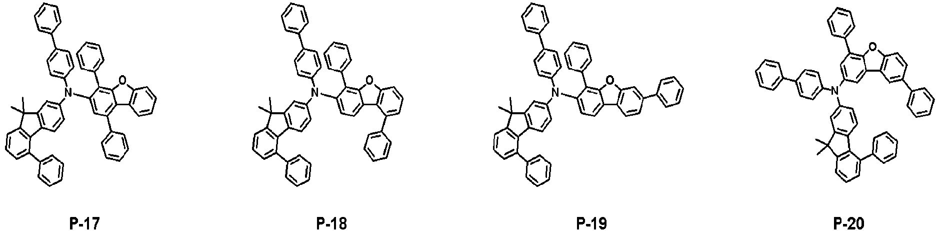

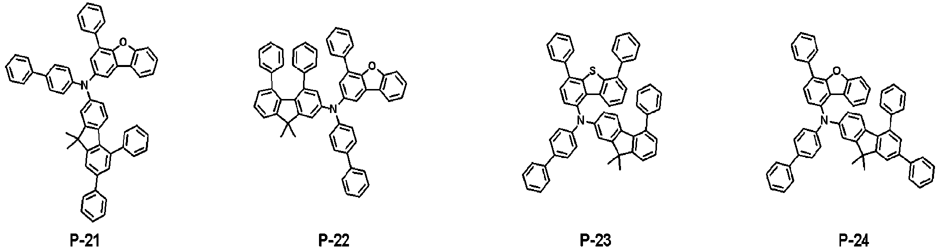

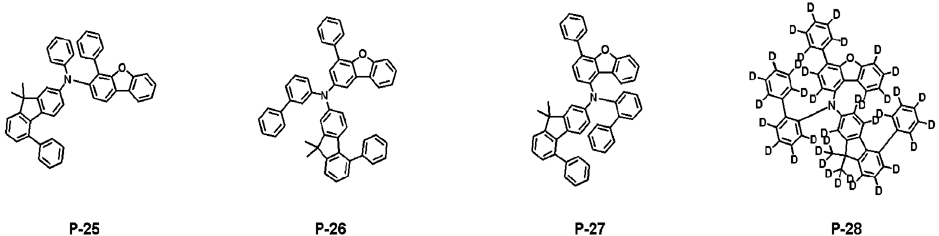

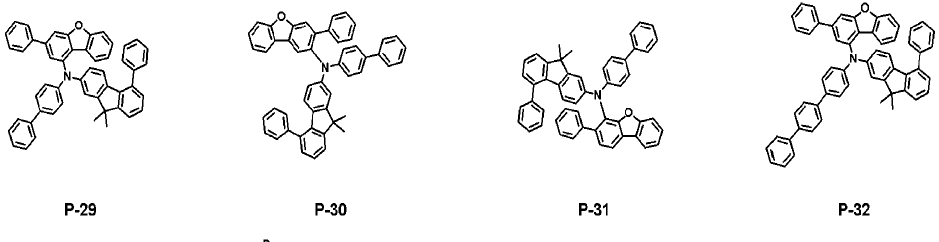

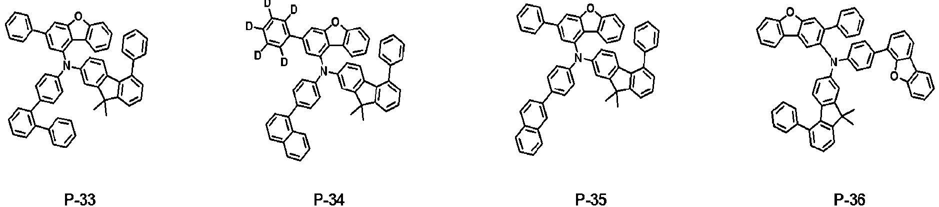

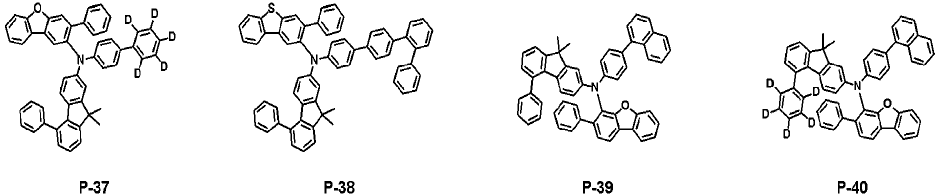

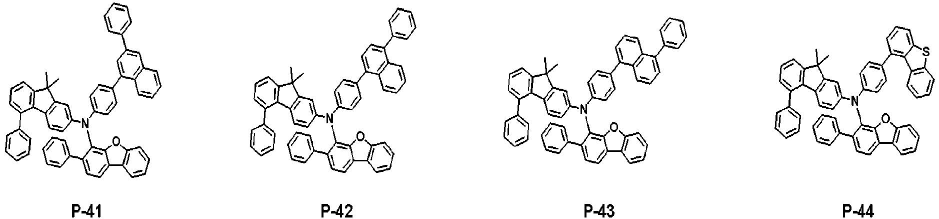

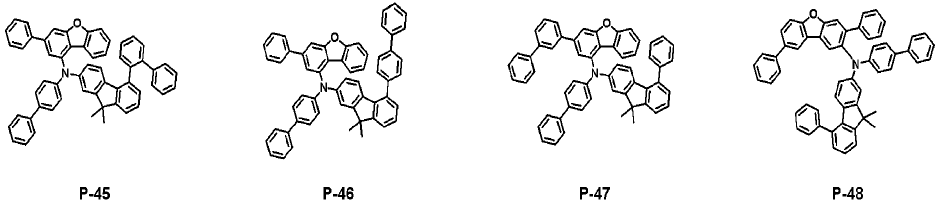

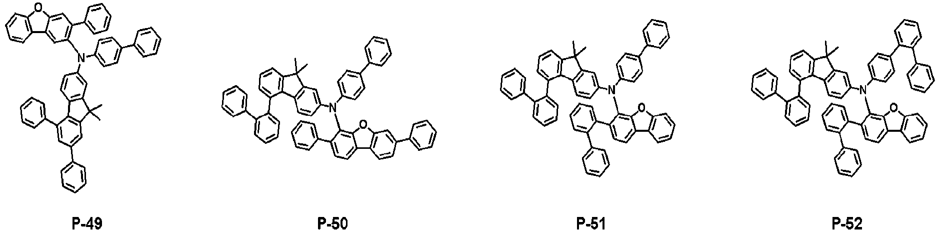

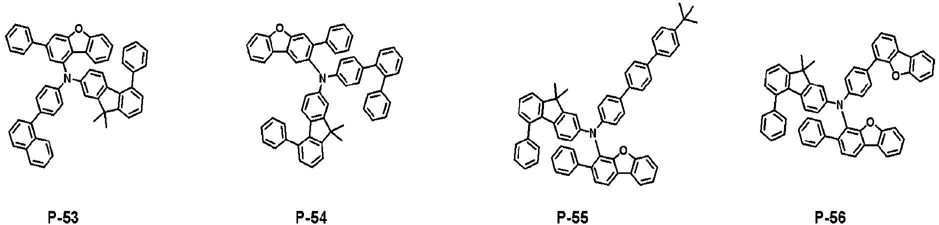

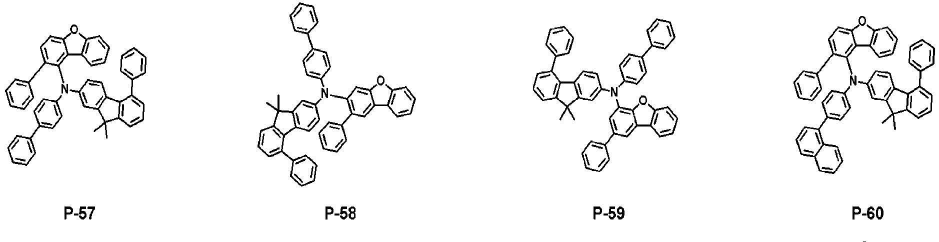

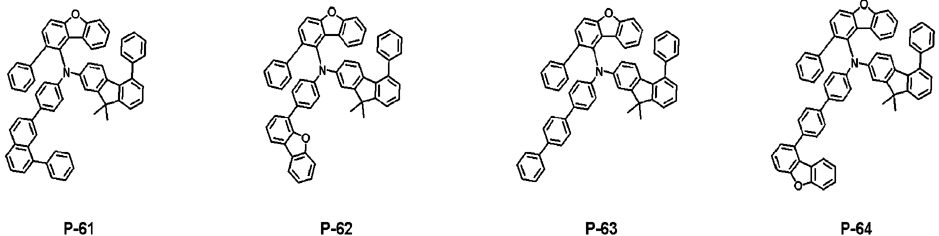

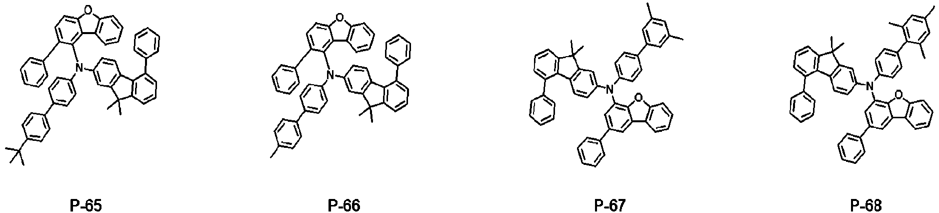

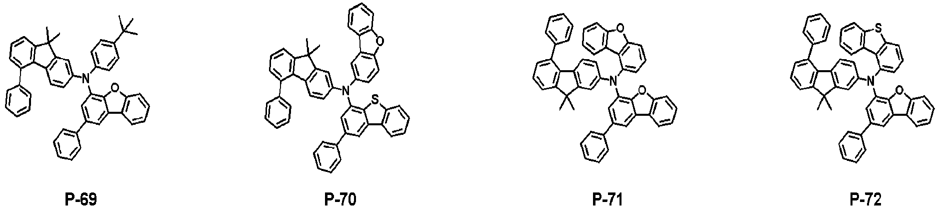

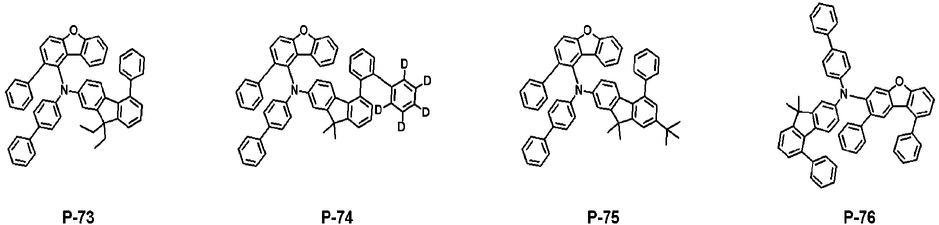

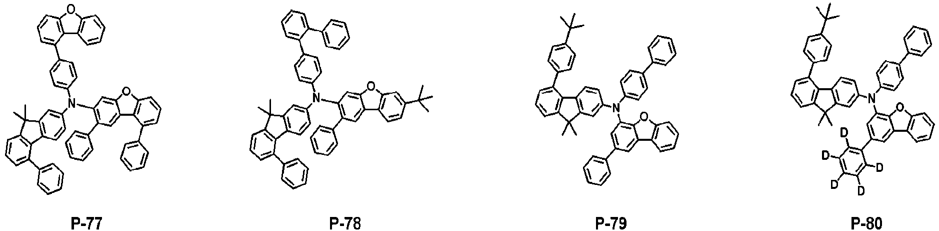

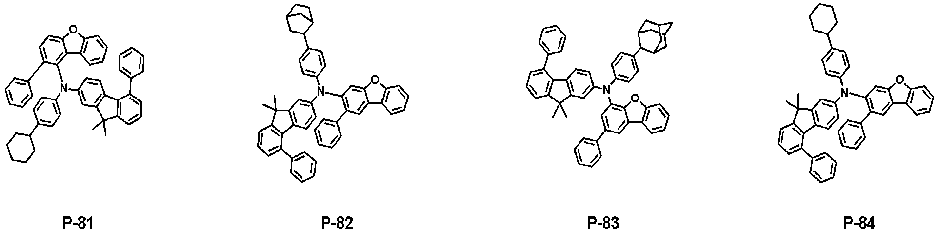

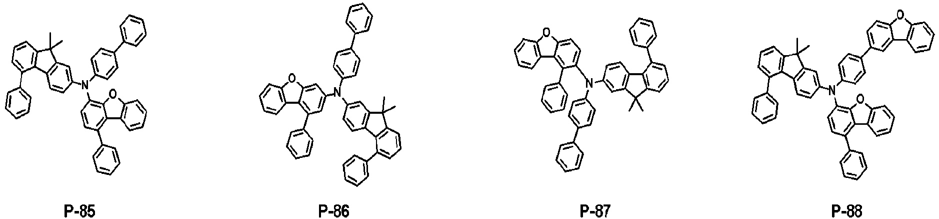

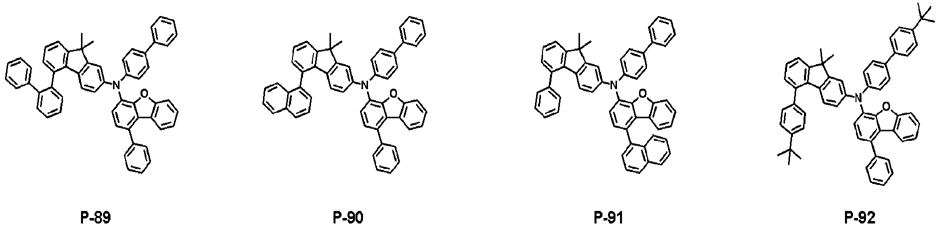

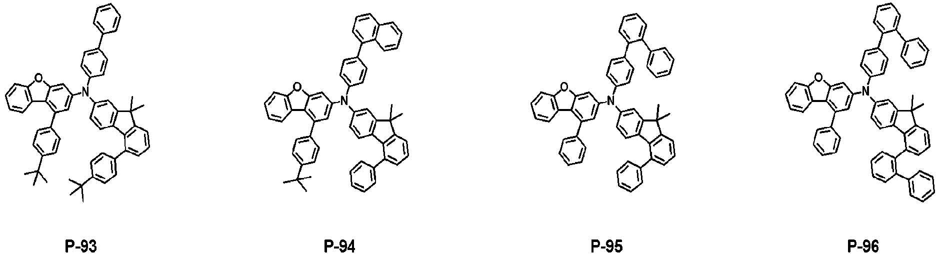

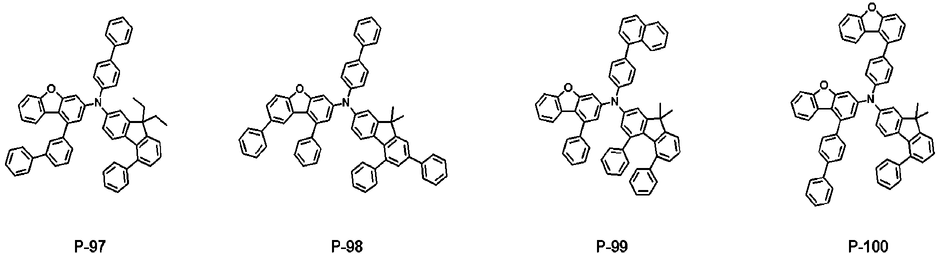

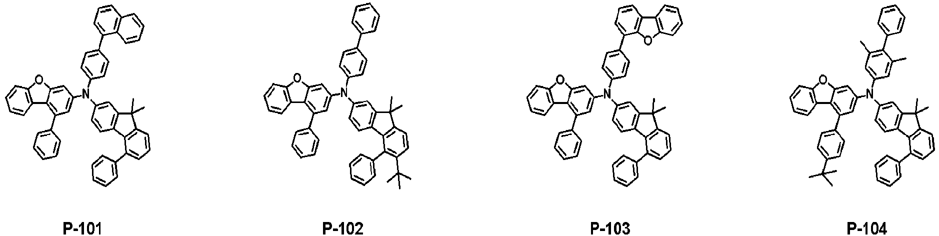

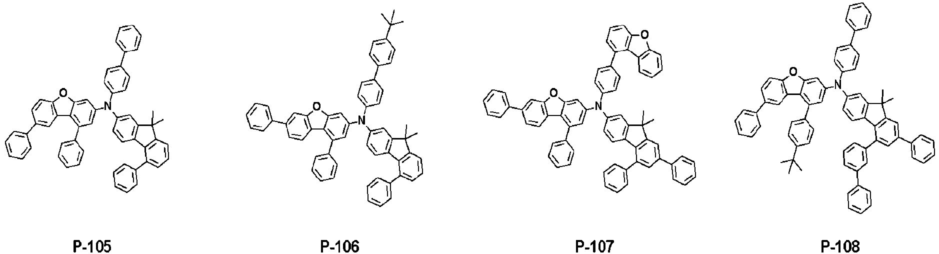

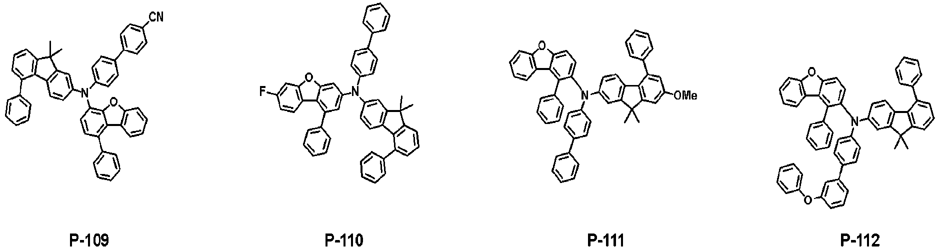

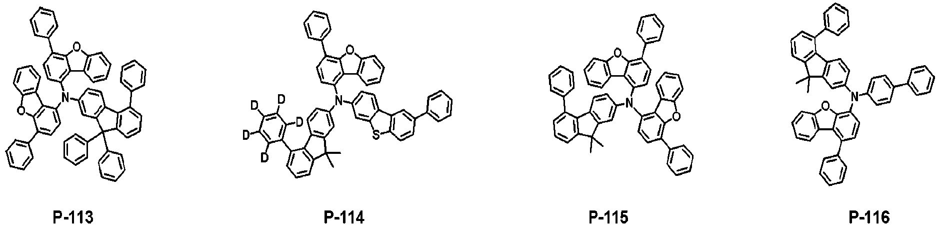

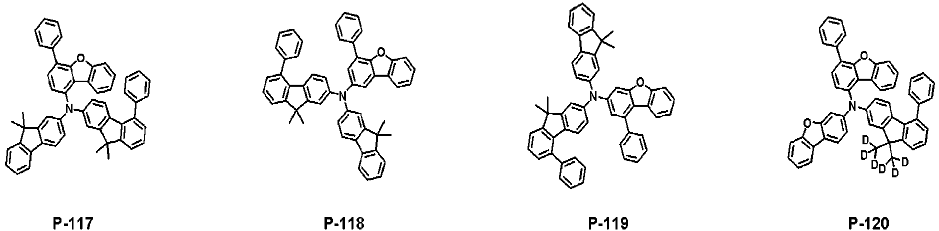

6. An organic electronic element comprising an anode; a cathode; and an organic material layer formed between the anode and the cathode; wherein the organic material layer comprises a single compound or 2 or more compounds represented by Formula (1) in claim 1.

7. The organic electronic element according to claim 6, wherein the organic material layer comprises at least one of a hole injection layer, a hole transport layer, an emitting-auxiliary layer, an emitting layer, an electron transport auxiliary layer, an electron transport layer, and an electron injection layer.

8. The organic electronic element according to claim 6, wherein the organic material layer is an emitting auxiliary layer.

9. The organic electronic element according to claim 6, further comprising a light efficiency enhancing layer formed on at least one surface of the anode and the cathode, the surface being opposite to the organic material layer.

10. The organic electronic element according to claim 6, wherein the organic material layer comprises 2 or more stacks comprising a hole transport layer, an emitting layer and an electron transport layer sequentially formed on the anode.

11. The organic electronic element according to claim 10, wherein the organic material layer further comprises a charge generation layer formed between the 2 or more stacks.

12. An electronic device comprising a display device comprising the organic electronic element of claim 6; and a control unit for driving the display device.

13. The electronic device according to claim 12, wherein the organic electronic element is at least one of an OLED, an organic solar cell, an organic photo conductor(OPC), organic transistor (organic TFT) and an element for monochromic or white illumination.

14. A method for reusing Formula (1) of claim 1 comprising:
recovering a crude organic light emitting material comprising Formula (1) of claim 1 from a deposition apparatus used in the process for depositing the organic emitting material to prepare an organic an organic light emitting device;
removing impurities from the crude organic light emitting material;
recovering the organic light emitting material after the impurities are removed; and
purifying the recovered organic light emitting material to have a purity of 99.9% or higher.
